(19)

**Europäisches Patentamt**
**European Patent Office**
**Office européen des brevets**

(11) **EP 3 052 940 B1**

(12) **EUROPEAN PATENT SPECIFICATION**

(45) Date of publication and mention
of the grant of the patent:
**26.05.2021   Bulletin 2021/21**

(51) Int Cl.:
***G01N 33/574*** *(2006.01)*

(21) Application number: **14849862.9**

(86) International application number:
**PCT/US2014/058304**

(22) Date of filing: **30.09.2014**

(87) International publication number:
**WO 2015/048740 (02.04.2015 Gazette 2015/13)**

(54) **GENOTYPIC AND PHENOTYPIC ANALYSIS OF CIRCULATING TUMOR CELLS TO MONITOR TUMOR EVOLUTION IN PROSTATE CANCER PATIENTS**

GENOTYPEN- UND PHÄNOTYPENANALYSE ZIRKULIERENDER TUMORZELLEN ZUR ÜBERWACHUNG DER TUMORENTWICKLUNG BEI PATIENTEN MIT PROSTATAKREBS

ANALYSE GÉNOTYPIQUE ET PHÉNOTYPIQUE DE CELLULES TUMORALES CIRCULANTES PERMETTANT DE SURVEILLER L'ÉVOLUTION D'UNE TUMEUR CHEZ DES PATIENTS ATTEINTS DE CANCER DE LA PROSTATE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:   **30.09.2013   US 201361884835 P**

(43) Date of publication of application:
**10.08.2016   Bulletin 2016/32**

(73) Proprietors:
 • **The Scripps Research Institute**
   **La Jolla, CA 92037 (US)**
 • **Cold Spring Harbor Laboratories**
   **Cold Spring Harbor, NY 11724 (US)**
 • **Kuhn, Peter**
   **Solana Beach, CA 92075 (US)**
 • **Dago Rodriguez, Angel Ernesto**
   **La Jolla, CA 92037 (US)**
 • **Carlsson, Anders**
   **San Diego, CA 92019 (US)**
 • **Liu, Wei**
   **San Diego, CA 92122 (US)**
 • **Hicks, Jim**
   **Lattingtown, NY 11560 (US)**

(72) Inventors:
 • **KUHN, Peter**
   **Solana Beach, CA 92075 (US)**
 • **DAGO RODRIGUEZ, Angel Ernesto**
   **La Jolla, CA 92037 (US)**

 • **CARLSSON, Anders**
   **Los Angeles, CA 90403 (US)**
 • **LIU, Wei**
   **Chandler, AZ 85225 (US)**
 • **HICKS, Jim**
   **Lattingtown, NY 11560 (US)**

(74) Representative: **Hutter, Anton et al**
   **Venner Shipley LLP**
   **5 Stirling House**
   **Stirling Road**
   **The Surrey Research Park**
   **Guildford GU2 7RF (GB)**

(56) References cited:
   **WO-A1-2015/116828      WO-A2-2012/103025**
   **WO-A2-2012/103025      US-A1- 2010 184 093**
   **US-A1- 2012 276 555      US-A1- 2013 171 642**

**(Cont. next page)**

- **D. T. MIYAMOTO ET AL: "Androgen Receptor Signaling in Circulating Tumor Cells as a Marker of Hormonally Responsive Prostate Cancer", CANCER DISCOVERY, vol. 2, no. 11, 23 October 2012 (2012-10-23), pages 995-1003, XP055211802, ISSN: 2159-8274, DOI: 10.1158/2159-8290.CD-12-0222 & D T Miyamoto ET AL: "Androgen Receptor Signaling in Circulating Tumor Cells as a Marker of Hormonally Responsive Prostate Cancer: Supplemental Methods Single Molecule Sequencing and AR transcriptional signature", , 1 November 2012 (2012-11-01), pages 1-3, XP055359620, Retrieved from the Internet: URL:http://cancerdiscovery.aacrjournals.or g/content/candisc/suppl/2012/09/14/2159-82 90.CD-12-0222.DC1/meth_49K.pdf [retrieved on 2017-03-28] & D T Miyamoto ET AL: "Androgen Receptor Signaling in Circulating Tumor Cells as a Marker of Hormonally Responsive Prostate Cancer: Supplemental Figure S1", Cancer Discovery, 1 Nov**
- **STOTT SHANNON L ET AL: "Isolation and Characterization of Circulating Tumor Cells from Patients with Localized and Metastatic Prostate Cancer", SCIENCE TRANSLATIONAL MEDICINE,, vol. 2, no. 25, 31 March 2010 (2010-03-31) , pages 111-120, XP009168515, ISSN: 1946-6234, DOI: 10.1126/SCITRANSLMED.3000403**
- **GERHARDT ATTARD ET AL: "Utilizing circulating tumor cells: challenges and pitfalls", CURRENT OPINION IN GENETICS & DEVELOPMENT, vol. 21, no. 1, 3 November 2010 (2010-11-03), pages 50-58, XP028357969, ISSN: 0959-437X, DOI: 10.1016/J.GDE.2010.10.010 [retrieved on 2010-11-03]**
- **GERHARDT ATTARD ET AL: "Selective Inhibition of CYP17 With Abiraterone Acetate Is Highly Active in the Treatment of Castration-Resistant Prostate Cancer", JOURNAL OF CLINICAL ONCOLOGY, vol. 27, no. 23, 10 August 2009 (2009-08-10), pages 3742-3748, XP055358352, US ISSN: 0732-183X, DOI: 10.1200/JCO.2008.20.0642**
- **V. Melnikova ET AL: "Molecular Characterization of Circulating Tumor Cells Using A Highly Sensitive Method of Enrichment Based on the CellSearch CTC Profile Kit", , 1 January 2010 (2010-01-01), XP055058480, 22nd EORTC - NCI-AACR Symposium on Molecular Targets and Cancer; poster session, Berlin DOI: 10.1016/S1359-6349(10)72333-8 Retrieved from the Internet: URL:http://www.apocell.com/wp-content/uplo ads/2010/12/EORTC-poster-2010.pdf [retrieved on 2013-04-04]**
- **DANILA ET AL.: 'TMPRSS2-ERG Status in Circulating Tumor Cells as a Predictive Biomarker of Sensitivity in Castration-Resistant Prostate Cancer Patients Treated With Abiraterone Acetate' EUROPEAN UROLOGY vol. 60, no. 5, 01 November 2011, pages 897 - 905, XP028300945**
- **JIANG ET AL.: 'Detection of Androgen Receptor Mutations in Circulating Tumor Cells in Castration-Resistant Prostate Cancer.' CLINICAL CHEMISTRY vol. 56, no. 9, 01 September 2010, pages 1492 - 1495, XP055330897**
- **MARRINUCCI ET AL.: 'Fluid Biopsy in Patients with Metastatic Prostate, Pancreatic and Breast Cancers' PHYSICAL BIOLOGY vol. 9, no. 1, 01 February 2012, pages 1 - 19, XP020218196**
- **MIYAMOTO ET AL.: 'Androgen Receptor Signaling in Circulating Tumor Cells as a Marker of Hormonally Responsive Prostate Cancer' CANCER DISCOVERY vol. 2, no. 11, 01 November 2012, pages 995 - 1003, XP055211802**

## Description

The invention relates generally to the field of cancer diagnostics and, more specifically to methods for predicting response to a hormone-directed therapy in a prostate cancer (PCa) patient.

## BACKGROUND

Prostate cancer (PCa) remains the most common non-cutaneous cancer in the US. In 2014 alone, the projected incidence of prostate cancer is 233,000 cases with deaths occurring in 29,480 men, making metastatic prostate cancer therapy truly an unmet medical need. Siegel et al., 2014. CA Cancer J Clin. 2014;64(1):9-29. Epidemiological studies from Europe show comparable data with an estimated incidence of 416,700 new cases in 2012, representing 22.8% of cancer diagnoses in men. In total, 92,200 PCa-specific deaths are expected, making it one of the three cancers men are most likely to die from, with a mortality rate of 9.5%

The androgen-androgen receptor (AR) signaling pathway is essential for the development and progression of prostate cancer and is a key target of many therapeutic agents. In metastatic prostate cancer (PCa), androgen deprivation therapy (ADT), constitutes the gold standard treatment to induce tumor regression by suppressing AR activation. Despite initial response to ADT, patients often develop resistance and progress to castration resistant prostate cancer (CRPC), an incurable disease with poor prognosis. These patients are often treated with salvage hormone-directed therapies, including agents such as non-steroidal anti-androgens and androgen-synthesis inhibitors . In managing these treatments, predicting therapeutic response and identifying early indicators of therapy resistance are major challenges. The levels of prostate specific antigen (PSA), an androgen regulated protein measured in the serum, is used to monitor therapeutic response in CRPC patients, however its predictive capability for this patient group is limited. In addition, while many studies have identified molecular events that may contribute to therapeutic resistance to androgen-targeting agents, it is difficult to apply these findings due to the limited supply of sequentially acquired tissue and the expected heterogeneity across multiple metastatic deposits present in any individual patient. As such, methods that would allow for non-invasive sequential monitoring through the clinical course of therapy would be of tremendous value to clinicians.

Circulating tumor cells (CTCs) have the potential to provide a non-invasive means of assessing progressive cancers in real time during therapy, and further, to help direct therapy by monitoring phenotypic physiological and genetic changes that occur in response to therapy. In most CRPC patients, the primary tumor has been removed, and CTCs are expected to consist of cells shed from metastases, providing a 'fluid biopsy'. Currently, the only method approved for CTC enumeration (Cell-Search, Veridex) is based on an immune enrichment approach that pre-selects for cells that express Epithelial Cell Adhesion Molecule (EpCAM), an epithelial cell surface marker. Although, numeric quantification of CTCs using CellSearch has yielded some prognostic information in certain cancers, this methodology has limitations such as low sensitivity (cells with low or absent EpCAM expression won't be captured) and the regular presence/contamination of genomically normal leukocytes in the sample preparation that hampers further molecular characterization and data interpretation. Recently, genomic changes based on array CGH and limited sequencing has been reported on CTCs isolated with the CellSearch system. Detailed analysis in paired tumors and metastasis (n = 2) and CTCs (n = 8) suggested that most mutations detected in CTCs were present at a low-level in the primary tumor. However, because a single timepoint during the clinical course of the disease was investigated this study does not address how a tumor may respond and evolve to therapeutic pressure.

Miyamoto et al, Cancer Discovery, vol. 2, no.11, October 2012, pages 995-1003, discloses microfluidic capture of CTCs to measure AR signalling readouts before and after therapeutic interventions. US 2013/0171642 discloses methods of automated characterisation of CTCs, for example using automated tissue strainers. Stott et al, Science Translational Medicine, vol. 2, no. 25, March 2010, pages 111-120, discloses a quantitative automated imaging system for analysis of prostate CTCs. Attard et al, Current Opinion in Genetics & Development, vol. 21, no. 1, November 2010, pages 50-58, discusses challenges around detecting CTCs in the blood of cancer patients. US 2012/0276555 discloses seminal computational approaches utilising data from non-rare cells to detect rare cells, such as CTCs. WO 2012/103025 discloses methods for identifying and obtaining CTCs from a patient's blood sample for further analysis. WO 2015/116828 discloses a method of predicting resistance to AR targeted therapy in a prostate cancer patient by identifying CTCs and determining the presence of a biomarker signature that is predictive of resistance to AR targeted therapy.

A need exists for diagnostic methods that provide a more comprehensive portrait of the molecular changes occurring, at the single cell level, in a CRPC patient under the treatment pressure in both ADT and chemotherapy settings to allow association of the emergence of distinct CTC subpopulations with the clinical course of the disease. The present invention addresses this need by enabling to trace over time the molecular changes in a patient's CTC population by correlating morphometric and protein expression data with genome wide CNV alterations for individual CTCs isolated at clinically significant timepoints. Related advantages are provided as well.

## SUMMARY OF THE INVENTION

The invention is as defined in the claims.

The present invention provides a method of predicting response to a hormone-directed therapy in a prostate cancer patient comprising

(a) performing a direct analysis of circulating tumour cells (CTCs) in the context of surrounding nucleated cells present in a blood sample obtained from the patient in the absence of enrichment of the blood sample for CTCs prior to detection thereof, comprising immunofluorescent staining and morphological characteristization of nucleated cells in the blood sample to identify and enumerate circulating tumour cells (CTC);

(b) individually characterizing genotypic, morphometric and protein expression parameters to generate a profile for each of the CTCs, wherein said genotypic parameters comprise copy number variation (CNV) signatures, wherein the CNV signatures comprise gene amplifications or deletions, wherein said gene amplifications comprise genes associated with androgen independent cell growth, wherein said gene comprises v-myc avian myelocytomatosis viral oncogene homolog (MYC);

(c) predicting response to hormone-directed therapy in the prostate cancer patient based on said profiles of the CTCs;

(d) repeating steps (a) through (c) at one or more timepoints after initial diagnosis of prostate cancer to sequentially monitor said genotypic, morphometric and protein expression parameters; and

(e) predicting a response based on comparison of the profiles between the timepoints.

In particular examples, the methods further comprise isolating the CTCs subsequent to the characterization of the morphometric and protein expression parameters and prior to the characterization of the genotypic parameters.

In some embodiments, the methods comprise repeating steps (a) through (c) at one or more timepoints after initial diagnosis of prostate cancer to sequentially monitor said genotypic, morphometric and protein expression parameters. In some embodiments the timepoints are at intervals that coincide with expected decision points in the standard care of CRPC. In some embodiments the timepoints coincide with clinical progression of the PCa.

In some embodiments, the method further comprises identifying clonal lineages of each CTC by genomic analysis. In additional embodiments, the cancer is metastatic castration resistant PCa (mCRPC). In some embodiments, the hormone directed therapy comprises Androgen Deprivation Therapy (ADT), which can be the first line or second line hormonal therapy. In some embodiments, the second line hormonal therapy blocks synthesis of androgen and is selected from the group consisting of abiraterone acetate, ketoconazole and aminoglutethimide.

In some embodiments the methods for predicting response to a hormone-directed therapy in a prostate cancer (PCa) patient, the immunofluorescent staining of nucleated cells comprises pan cytokeratin, cluster of differentiation (CD) 45, diamidino-2-phenylindole (DAPI) and androgen receptor (AR).

In some examples of the disclosed methods for predicting response to a hormone-directed therapy or chemotherapy in a prostate cancer (PCa) patient, the genotypic parameters comprise genomic variations including, for example, structural variations (SVs) and copy number variations (CNVs), simple nucleotide variations (SNVs), including single-nucleotide polymorphisms (SNPs) and small insertions and deletions (INDELs). In particular embodiments of the methods for predicting response to a hormone-directed therapy or chemotherapy in a prostate cancer (PCa) patient, the genotypic parameters comprise copy number variation (CNV) signatures. In some embodiments, CNVs are gene amplifications or deletions. In further embodiments, the gene amplifications comprise genes associated with androgen independent cell growth, for example, AR or v-myc avian myelocytomatosis viral oncogene homolog (MYC). In some examples, the genotypic parameters are detected by next generation sequencing (NGS).

In some embodiments the methods for predicting response to a hormone-directed therapy or chemotherapy in a prostate cancer (PCa) patient, the protein expression parameters comprise quantifying protein expression level or subcellular localization of protein expression. In further examples, the protein expression level is quantified by measuring strength of immunofluorescent signal using high resolution immunofluorescence imaging. In particular embodiments, the protein expression is AR expression. In some embodiments the methods for predicting response to a hormone-directed therapy in a prostate cancer (PCa) patient, the morphometric parameters comprise cell shape.

In some embodiments of the disclosed methods for predicting response to a hormone-directed therapy or chemotherapy in a prostate cancer (PCa) patient, the response is predicted based on comparison of the profiles between the timepoints. In some embodiments of the disclosed methods for predicting response to a hormone-directed therapy or chemotherapy in a prostate cancer (PCa) patient, the predicted response is emergence of resistant disease. In certain embodiments, the identification of a resistant CTC correlates with the emergence of resistant disease and the resistant CTC represents a clonal lineage that predominates resistant disease. In some emdiments, the re-emergence of AR positive CTCs predicts emergence of resistant disease. In further embodiments, the re-emergence of the AR positive CTCs is accompanied by genomic alterations such as AR or MYC amplification. In some embodiments, the re-emergence of AR positive CTCs is accompanied by morphometric change such as a decrease in cell roundness. In particular examples, the methods include determining whether a resistant CTC is AR independent, AR ligand independent or both. In some examples, the determination of whether a resistant CTC is AR independent, AR ligand independent or both informs a subsequent therapy selection or therapy change, for example, if the resistant cell is AR positive, the patient is a candidate for AR targeted treatment despite being AR ligand independent.

In some examples of the disclosed methods for predicting response to a hormone-directed therapy or chemotherapy in a prostate cancer (PCa) patient, the isolation of the CTCs involves relocation from initial image acquisition, re-imaging of the CTCs and physical extraction of the CTCs.

Other features and advantages of the invention will be apparent from the detailed description, and from the claims.

**BRIEF DESCRIPTION OF THE DRAWINGS**

The patent or application file contains at least one drawing executed in color. Copies of this patent or patent application publication with color drawing(s) will be provided by the Office upon request and payment of the necessary fee.

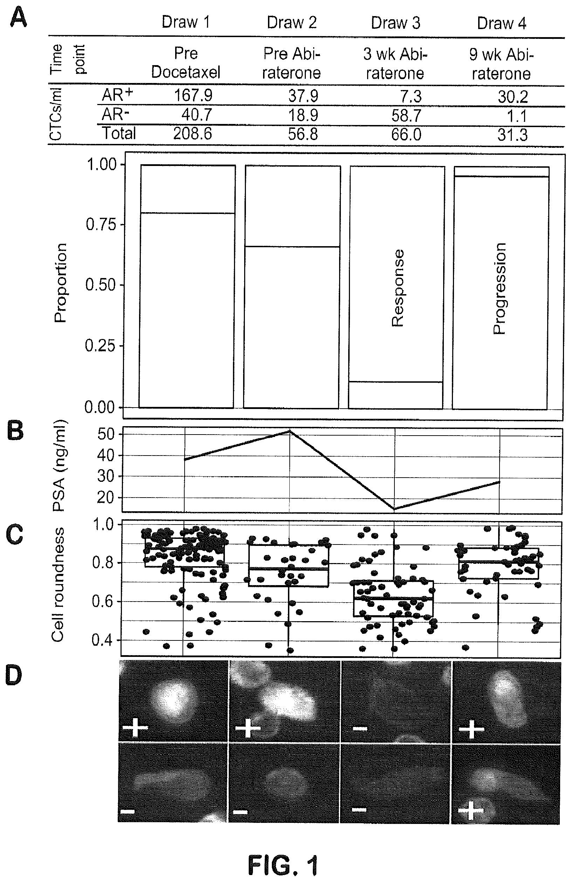

**Figure 1. Abiraterone acetate induces phenotypic alterations in the CTC population.** Panel (A) The total HD-CTCs counts, including the number of phenotypically distinct AR+ and AR2 cells, was determined for each blood Draw collected during therapeutic intervention. CTCs were defined as AR positive if the AR signal intensity was higher than six standard deviations over the mean (SDOM) of the surrounding leukocytes (background). The bar-graph shows the change in the distribution of the AR+ and AR2 CTC subpopulations along the course of treatment, indicated in red and blue respectively, and the numbers are presented above each bar. Panel (B) PSA concentration measured at each treatment timepoint. Panel (C) Boxplot of cell roundness for each individual CTC identified across the different treatment timepoints. Panel (D) Representative 40x immunofluorescence images of AR+ and AR2 HD-CTCs from the subpopulations identified in each treatment timepoint. Immunofluorescence channels are colored as follows: nucleus: blue; cytokeratin: red; AR: white; and CD45: green. AR phenotype is indicated in the bottom left corner of each image. All graphs were constructed using the ggplot2 and rgl packages in R.

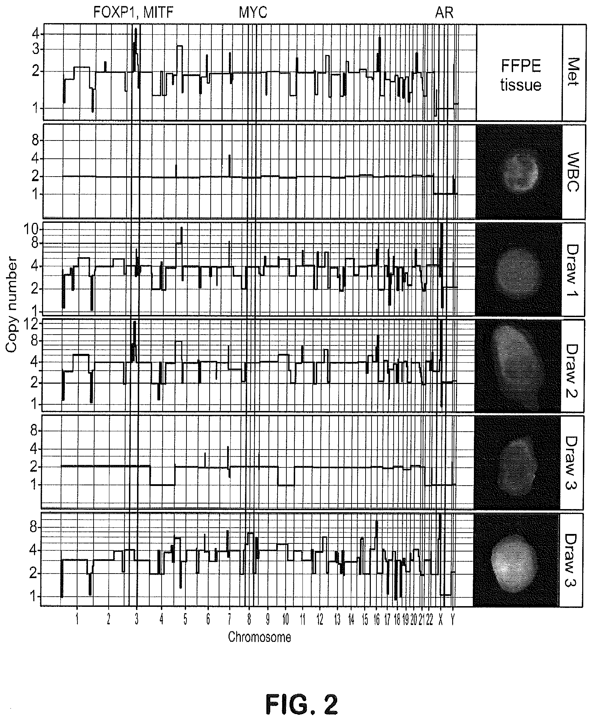

**Figure 2. Concurrent phenotypic and genotypic profiling of single prostate tumor cells.** Copy number variation profiles from the patient's bone metastasis; a control single WBC; and single CTCs from each of the four treatment timepoints are shown. The corresponding fluorescent image of the cell used to generate the CNV profile is shown to the right. Relevant genomic alterations and their chromosome localizations occurring in each specific draw are indicated with pale blue bars.

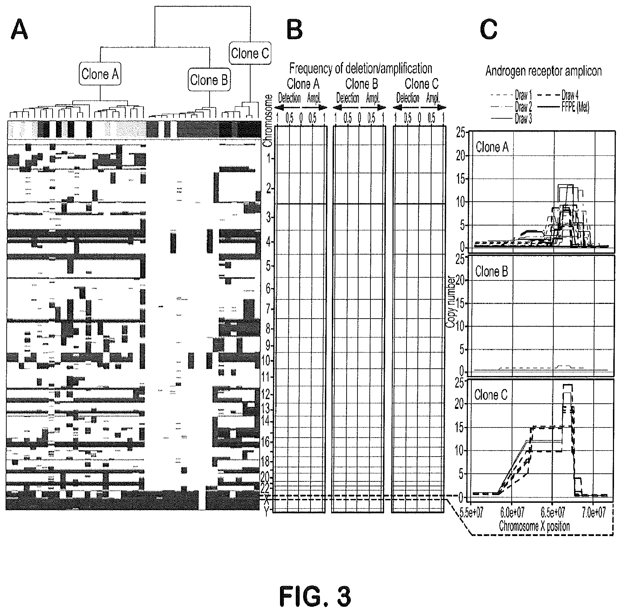

**Figure 3. Clonality and genomic aberrations in the CTC population.** Panel (A) Three different clonal lineages, represented as Cluster A, B and C, were identified based on the comparison of 41 single cell CNV profiles in an unsupervised hierarchical clustering. The blood draw from which each cell was isolated is indicated as Draw 1: yellow; Draw 2: orange; Draw 3: purple; and Draw 4: black. For reference, the bone metastasis FFPE tissue was included in the tree, colored in green. Below the tree, a heatmap indicates the amplifications (red) and deletions (blue) across the entire genome of each individual cell. Panel (B) Frequency of genomic amplifications and deletions in the three clusters identified. Areas uniquely amplified (red) or deleted (blue) in cluster A and C are highlighted. Panel (C) A detail plot of the AR amplification event colored per draw for each individual cluster is shown.

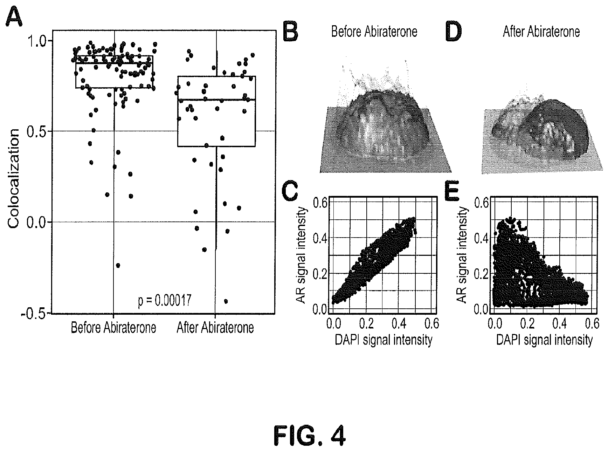

**Figure 4. AR subcellular localization changes at the time of disease progression.** Panel (A) Comparison of the AR subcellular localization in the CTCs identified in the blood prior to and after nine weeks of abiraterone treatment. Correlation between the AR and DAPI signals within the cell is indicative of AR being colocalized with DAPI, i.e. localized in the cell nucleus. High correlation was generally seen before abiraterone treatment, but a shift to less nuclear stain was observed after nine weeks of treatment (p = 0.00017, Wilcoxon sum-rank test). Panel (B) and Panel (D) Height maps constructed from the pixel intensities of CK (red), AR (green) and DAPI (blue) in representative CTCs to visualize the subcellular localization of AR. The cell in Panel (B) was isolated before abiraterone initiation and displays AR staining confined to the nucleus, while cytoplasmic AR staining is observed in the CTC identified at the time of therapeutic relapse Panel (D). Panel (C) and Panel (E) Plots of AR versus DAPI signal intensities for each pixel inside the cell in the 406images of the CTCs in Panel (B) and Panel (D), respectively. Each plot point is colored by the corresponding CK signal intensity. Nuclear localization was observed as positive correlation between the two intensities Panel (C), and nuclear exclusion as negative correlation Panel (E). All graphs and were done using the ggplot2 and rgl packages in R.

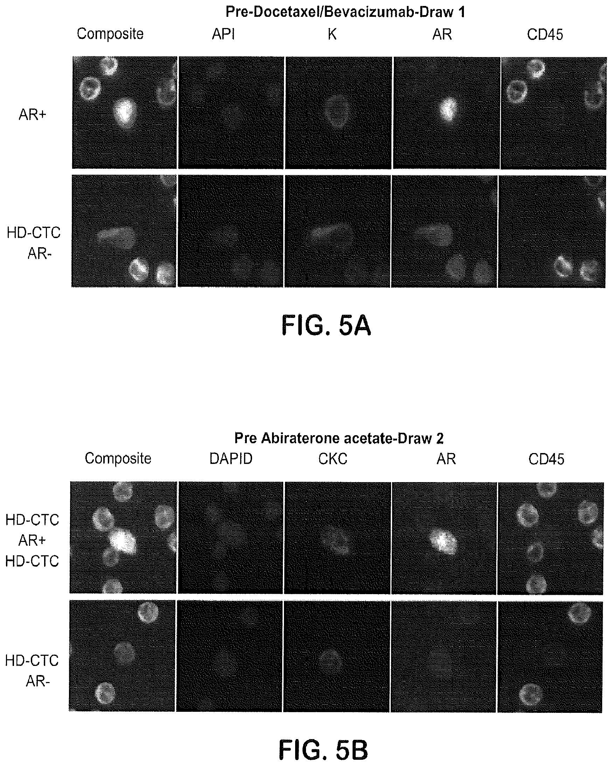

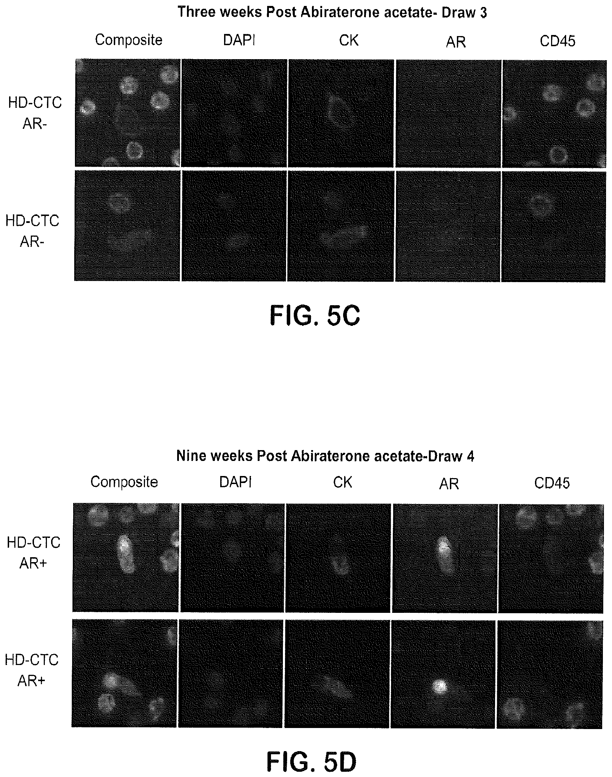

**Figure 5. Representative gallery of 40x high resolution immunofluorescence images of the two phenotypically distinct CTCs subpopulations identified.** A and B, Composite and non-merged images of an AR+ and AR- HD-CTC isolated from pre docetaxel (A) and pre abiraterone (B) treatment timepoints. C and D, Two different AR- and AR+ HD-CTCs, the predominant tumor cell phenotypes found in 3 (C) and 9 weeks post abiraterone (D). Panel D, CTCs with different pattern of AR subcellular localization. Nuclear and cytoplasmic AR is shown in the top panel and nuclear AR in the bottom panel. Composite and non-merged images for the individual immunofluorescence channels were colored as followed: DAPI (blue); cytokeratin-CK (red), androgen receptor-AR (white) and CD45 (green).

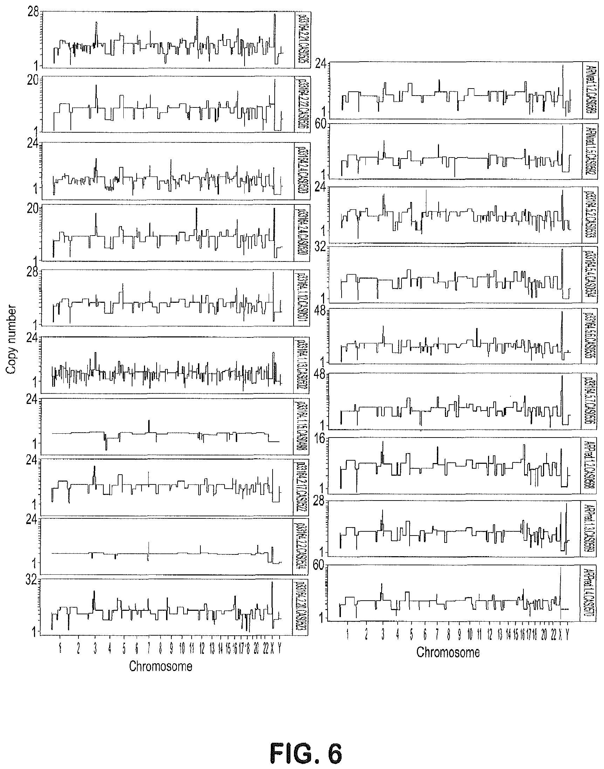

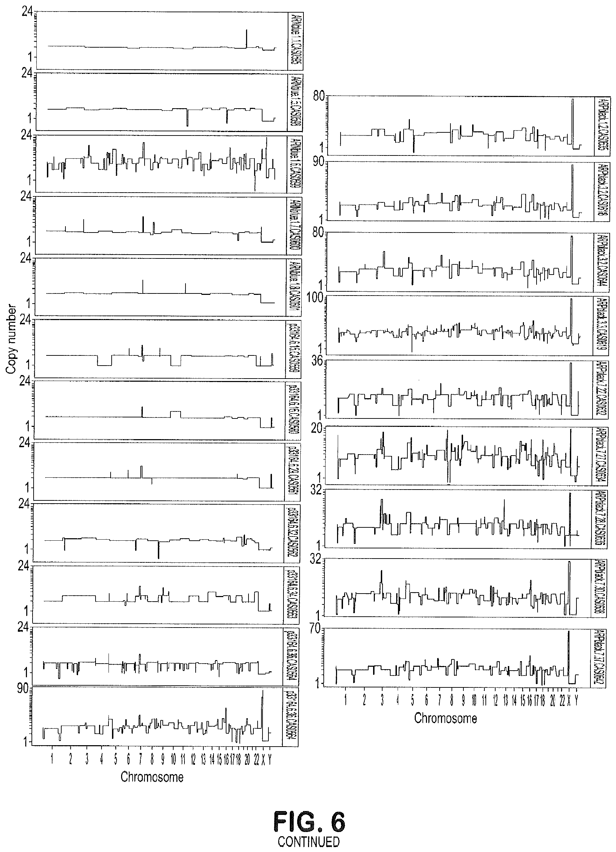

**Figure 6. Complete collection of single CTC CNV profiles.** The genome wide copy number fingerprints for all successfully profiled cells at each of different treatment timepoint.

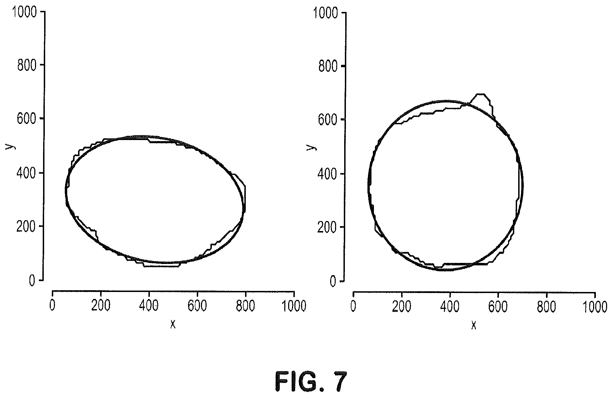

**Figure 7. Examples of cell roundness estimation.** The cell shape was analyzed by tracing the cell cytoplasm contour in the composite image of each CTC. The traced cell image was imported into R, and an ellipse was fitted to the shape using a least squares fitting algorithm described by Halir and Flusser, Proceeding of International Conference in Central Europe on Computer Graphics, Visualization and Interactive Digital Media: 125-132 (1998). Black line represents the manually drawn cell outline, red line the fitted ellipse. The cell roundness is estimated as the fraction of the *de facto* cell area and the area of a circle with the radius set to the cell's major axis. The cell roundness calculated to be 0.62 for the oval-shaped cell (left) and 0.96 for the more rounded cell (right). The p-value used in the comparison of the roundness between the CTCs isolated between the different draws was calculated using the Wilcoxon rank-sum test.

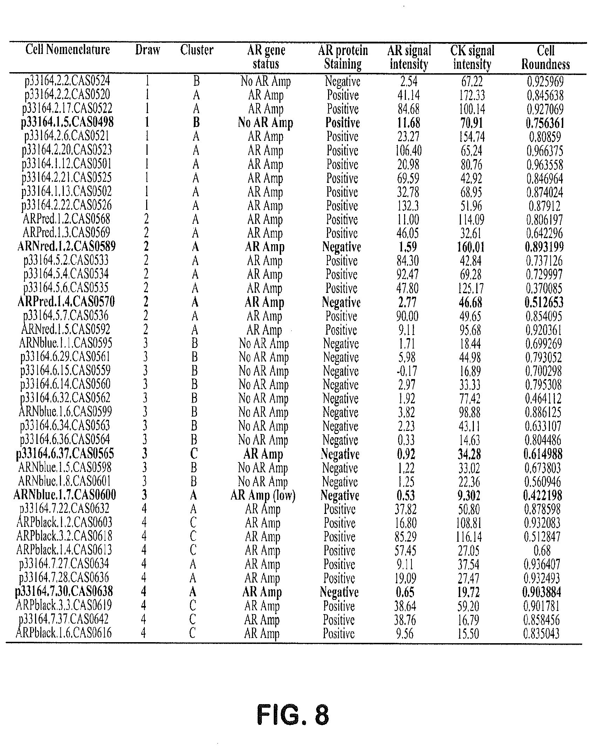

**Figure 8. Summary of the different phenotypic and genotypic traits analyzed in the 41 individual cells profiled for copy number alterations.** Concordance between AR phenotype-genotype was determined by comparison of the AR amplification status with the AR staining phenotype (Negative or Positive) for each individual cell. In red are cells that exhibited discordant AR phenotype-genotype.

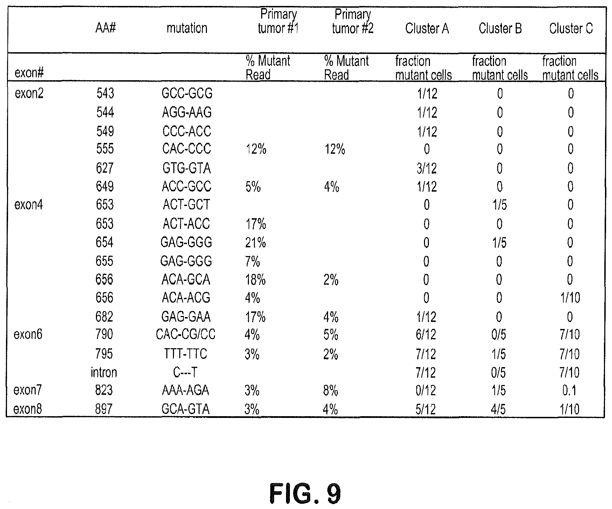

**Figure 9.** Table showing single nucleotide variants (SNV) in exons and introns of the Androgen Receptor (AR) gene in two blocks of the primary tumor and in circulating cells from patient JH33164. Clusters A,B,C represent distinct lineages of circulating cells based on copy number (CNV) profiling by NextGen sequencing. Direct sequencing of amplified DNA from each single cell was performed using targeted PCR primers to amplify each exon of the AR gene, followed by multiplex sequencing using barcoded Illumina sequencing adaptors.

## DETAILED DESCRIPTION

**The present disclosure is based, in part, on the achievement of correlating genomic events with complex phenotypic alterations at single cell level with time resolution in CTCs of a cancer patient. Significantly, the methods disclosed herein enable detection of the emergence of distinct CTC subpopulations endowed with specific molecular alterations along the clinical course of the disease. Based on the detection of these distinct CTC subpopulations characterized by genotypic, morphometric and protein expression alterations, the methods disclosed herein enable the prediction of resistance and clinical escape in a prostate cancer patient undergoing targeted hormone therapy and allow for clinical intervention.

**The present disclosure is based on the ability to capture the molecular changes in the CTC population by correlating morphometric and protein expression data with genome wide CNV alterations for individual CTCs isolated at clinically significant timepoints.

**As disclosed herein, the invention provides novel methods to achieve a comprehensive portrait of the molecular changes occurring, at the single cell level, in a CRPC patient under the treatment pressure in both ADT and chemotherapy settings. The High Definition-CTC (HD-CTC) method was used for the longitudinal identification and enumeration of CTCs (Marrinucci et al., Phys Biol 9: 016003 (2012)) and to asses for the expression of the AR. Lazar et al., Phys Biol 9: 016002 (2012). The methods employ an unbiased protocol to examine and distinguish CTCs among the surrounding leukocytes based on their cytokeratin positive (CK+) phenotype by using a high resolution immunofluorescence imaging. In addition, the HD-CTC technology preserves the cell morphology in such a way that enables the morphometric and the indirect quantification of AR and CK protein expression levels for all the CTCs identified in the blood sample. To further characterize each CTC, a protocol was developed for extracting individual cells under conditions suitable for subsequent genomic analysis by a modification of the single nucleus sequencing method described by Navin et al., Nature 472: 90-94 (2011), and Baslan et al., Nat Protoc 7: 1024-1041 (2012).

**A fundamental and enabling aspect of the present disclosure is the unparalleled robustness of the disclosed methods with regard to the detection of CTCs. The rare event detection disclosed herein with regard to CTCs is based on a direct analysis, *i.e.* non-enriched, of a population that encompasses the identification of rare events in the context of the surrounding non-rare events. Identification of the rare events according to the disclosed methods inherently identifies the surrounding events as non-rare events. Taking into account the surrounding non-rare events and determining the averages for non-rare events, for example, average cell size of non-rare events, allows for calibration of the detection method by removing noise. The result is a robustness of the disclosed methods that cannot be achieved with methods that are not based on direct analysis, but that instead compare enriched populations with inherently distorted contextual comparisons of rare events. The robustness of the direct analysis methods disclosed herein enables identification, enumeration and characterization of HD-CTCs, including subtypes of CTCs described herein, that enables the morphometric and the indirect quantification of AR and CK protein expression levels for all the CTCs identified in the blood sample that cannot be achievied with other CTC detection methods and that enables the analysis of correlation of genotypic and phenotypic changes in the context of the claimed methods.

**The rapid evolution of drug therapies in prostate cancer has vastly improved upon the use of docetaxel since its pivotal US Food and Drug Administration (FDA) approval in 2004 and has brought about a new era where progress has been made beyond the use of androgen deprivation therapy (ADT) with the addition of novel hormonal agents, immunotherapy, second-line chemotherapy as well as radiopharmaceuticals. The choice of sequencing currently relies on patient profiles, whether symptoms of metastatic disease exist or not. While survival outcomes are undeniably improved with the use of these therapies, disease will ultimately progress on each regimen.

Androgens in the form of testosterone or the more potent dihydrotestosterone (DHT) have been well-defined drivers of progression of prostate cancer and differentiation of the prostate gland. As such, the backbone of treatment for advanced prostate cancers was established decades ago when castration in the form of surgical orchiectomy achieved significant prostate tumor regression. Since then, substitution to chemical castration has been employed mostly due to patient preference. ADT has therefore become the standard systemic treatment for locally advanced or metastatic prostate cancer. While ADT is almost always effective in most patients, disease progression to castration resistance inevitably occurs. It is now increasingly recognized that the androgen receptor (AR) remains overexpressed despite seemingly castrate levels of testosterone, since alternative receptors may activate the AR or other target genes may help perpetuate the castrate-resistant phenotype, hence the term "castration-resistance" has become widely adopted in the literature. The enhanced understanding of the role of these androgens in stimulating the growth of prostate cancer has led to the development and approval of both abiraterone and enzalutamide.

Chemotherapy treatment uses drugs to attack cancerous cells directly or indirectly, with the aim of destroying cancer cells or slow their growth. Chemotherapy for prostate cancer can be recommended if a patient is not responding to hormonal therapy and the cancer has spread outside the prostate. Chemotherapy is the use of drugs to destroy cancer cells, usually by stopping their ability to grow and divide. Systemic chemotherapy is delivered through the bloodstream to reach cancer cells throughout the body. Chemotherapy for prostate cancer can help patients with advanced or castration-resistant prostate cancer.

It must be noted that, as used in this specification and the appended claims, the singular forms "a", "an" and "the" include plural referents unless the content clearly dictates otherwise. Thus, for example, reference to "a CTC" includes a mixture of two or more CTCs, and the like.

The term "about," particularly in reference to a given quantity, is meant to encompass deviations of plus or minus five percent.

As used in this application, including the appended claims, the singular forms "a," "an," and "the" include plural references, unless the content clearly dictates otherwise, and are used interchangeably with "at least one" and "one or more."

As used herein, the terms "comprises," "comprising," "includes," "including," "contains," "containing," and any variations thereof, are intended to cover a non-exclusive inclusion, such that a process, method, product-by-process, or composition of matter that comprises, includes, or contains an element or list of elements does not include only those elements but can include other elements not expressly listed or inherent to such process, method, product-by-process, or composition of matter.

The term "patient," as used herein preferably refers to a human, but also encompasses other mammals. It is noted that, as used herein, the terms "organism," "individual," "subject," or "patient" are used as synonyms and interchangeably.

As used herein, the term "circulating tumor cell" or "CTC" is meant to encompass any rare cell that is present in a biological sample and that is related to prostate cancer. CTCs, which can be present as single cells or in clusters of CTCs, are often epithelial cells shed from solid tumors found in very low concentrations in the circulation of patients.

As used herein, a "HD-CTC" refers to a single CTC that is cytokeratin positive, CD45 negative, contains a DAPI nucleus, and is morphologically distinct from surrounding white blood cells.

As used herein, "HD-CTC analysis" or "HD-SCA" (high definition single cell analysis) refers to analysis of any CTC based on genotypic, morphometric and protein expression parameters to generate a profile for each of the CTCs. High definition in the context of CTC and SC analysis therefore refers to high content analysis of all CTCs or rare cells present in a sample and is not limited to analysis of HD-CTCs.

In one embodiment, the disclosure provides a method of predicting response to a hormone-directed therapy in a prostate cancer (PCa) patient comprising (a) performing a direct analysis comprising immunofluorescent staining and morphological characteristization of nucleated cells in a blood sample obtained from the patient to identify and enumerate circulating tumor cells (CTC); (b) individually characterizing genotypic, morphometric and protein expression parameters to generate a profile for each of the CTCs, and (c) predicting response to hormone-directed therapy in the prostate cancer PCa patient based on the profile.

In one example, the disclosure provides a method of predicting response to chemotherapy in a prostate cancer (PCa) patient comprising (a) performing a direct analysis comprising immunofluorescent staining and morphological characteristization of nucleated cells in a blood sample obtained from the patient to identify and enumerate circulating tumor cells (CTC); (b) individually characterizing genotypic, morphometric and protein expression parameters to generate a profile for each of the CTCs, and (c) predicting response to chemotherapy in the prostate cancer PCa patient based on the profile.

In a further embodiment, the disclosure provides a method of predicting response to a hormone-directed therapy in a prostate cancer (PCa) patient comprising (a) performing a direct analysis comprising immunofluorescent staining and morphological characteristization of nucleated cells in a blood sample obtained from the patient to identify and enumerate circulating tumor cells (CTC); (b) individually characterizing genotypic, morphometric and protein expression parameters to generate a profile for each of the CTCs; (c) identifying clonal lineages of each CTC based on genomic analysis, (d) assigning each CTC to a clonal lineage, and (e) predicting response to hormone-directed therapy in the prostate cancer PCa patient based on a combination the profile and the clonal lineage.

In a further example, the disclosure provides a method of predicting response to chemotherapy in a prostate cancer (PCa) patient comprising (a) performing a direct analysis comprising immunofluorescent staining and morphological characteristization of nucleated cells in a blood sample obtained from the patient to identify and enumerate circulating tumor cells (CTC); (b) individually characterizing genotypic, morphometric and protein expression parameters to generate a profile for each of the CTCs; (c) identifying clonal lineages of each CTC based on genomic analysis, (d) assigning each CTC to a clonal lineage, and (e) predicting response to chemotherapy in the prostate cancer PCa patient based on a combination the profile and the clonal lineage.

In some examples, the methods further comprise isolating the CTCs subsequent to the characterization of the morphometric and protein expression parameters and prior to the characterization of said genotypic parameters. In some examples, the methods of the invention include an initial step of providing or obtaining a blood sample from the patient.

In metastatic prostate cancer (PCa), androgen deprivation therapy (ADT), constitutes the gold standard treatment to induce tumor regression by suppressing AR activation. ADT can include luteinizing hormone-releasing hormone (LHRH) agonists approved to treat prostate cancer including, for example, leuprolide, goserelin, and buserelin. Despite initial response to ADT, patients often develop resistance and progress to castration resistant prostate cancer (CRPC), an incurable disease with poor prognosis. These patients are often treated with salvage hormone-directed therapies, including agents such as non-steroidal anti-androgens and androgen-synthesis inhibitors. In some embodiments, the cancer is metastatic castration resistant PCa (mCRPC). In additional embodiments, the hormone directed therapy comprises Androgen Deprivation Therapy (ADT). In a further embodiment, the ADT is a second line hormonal therapy. In further embodiments, the second line hormonal therapy blocks synthesis of androgen and is selected from the group consisting of abiraterone acetate, ketoconazole and aminoglutethimide. Abiraterone acetate (Zytiga; Janssen Biotech, Inc. Horsham, PA, USA) is an FDA-approved inhibitor of androgen biosynthesis, which blocks cytochrome P450-c17 (CYP17), leading to suppression of androgens derived from the adrenal glands, the prostate tumor and the tumor microenvironment.

The method of predicting response to a hormone-directed therapy in a prostate cancer (PCa) patient disclosed herein comprise performing a direct analysis comprising immunofluorescent staining and morphological characteristization of nucleated cells in a blood sample obtained from the patient to identify and enumerate circulating tumor cells (CTC). As used herein in the context of generating CTC data, the term "direct analysis" means that the CTCs are detected in the context of all surrounding nucleated cells present in the sample as opposed to after enrichment of the sample for CTCs prior to detection. In some examples, the methods comprise microscopy providing a field of view that includes both CTCs and at least 200 surrounding white blood cells (WBCs). The lack of enrichment of the disclosed methods enables an unbiased approach to examine and distinguish CTCs among the surrounding leukocytes based on their cytokeratin positive (CK+) phenotype by using a high resolution immunofluorescence imaging. In addition, the HD-CTC technology described herein preserves the cell morphology in such a way that enables the morphometric and the indirect quantification of AR and CK protein expression levels for all the CTCs identified in the blood sample. Further enabling the present methods is the ability to extract individual cells under conditions suitable for subsequent genomic analysis as disclosed herein. As described further below, the immunofluorescent staining of nucleated cells comprises pan cytokeratin, cluster of differentiation (CD) 45, diamidino-2-phenylindole (DAPI) and androgen receptor (AR).

In some embodiments, CTCs are individually characterized based on genotypic, morphometric and protein expression parameters to generate a profile for each of the CTCs. In some examples of the disclosed methods for predicting response to a hormone-directed therapy or chemotherapy in a prostate cancer (PCa) patient, the genotypic parameters comprise genomic variations including, for example, structural variations (SVs) and copy number variations (CNVs), simple nucleotide variations (SNVs), including single-nucleotide polymorphisms (SNPs) and small insertions and deletions (INDELs). In particular embodiments, genotypic parameters used include detection of copy number variation (CNV) signatures, including genomic amplifications and examples. In further examples, the genotypic parameters measured comprise the number of genomic variations detected and/or the speed of occurrence of new genomic alterations. In some examples, the genomic amplifications and deletions affect regions containing oncogenes or genes implicated in the AR signaling axis. In further embodiments, gene amplifications include genes associated with androgen independent cell growth, for example, AR and v-myc avian myelocytomatosis viral oncogene homolog (MYC). In some examples, the genotypic parameters are detected by next generation sequencing (NGS). It will be understood by those skilled in the art, that he sequence analysis used in the methods of the invention can employ any useful sequencing technology, including without limitation amplification, polymerase chain reaction (PCR), real-time PCR (qPCR; RT-PCR), Sanger sequencing, next generation sequencing, restriction fragment length polymorphism (RFLP), pyrosequencing, DNA methylation analysis, or a combination thereof.

In some embodiments, protein expression parameters useful in practicing the methods disclosed herein include quantifying protein expression level and subcellular localization of protein expression. In some examples, the protein expression level is indirectly quantified by measuring strength of immunofluorescent signal using high resolution immunofluorescence imaging.

In some embodiments, the morphometric parameters useful in practicing the methods disclosed herein include cell shape, in particular, cell roundness. The cell shape (cell roundness) can be analyzed by tracing the cell cytoplasm contour in the composite image of each CTC. The traced cell image canbe imported into R, and an ellipsis was fitted to the shape using a least squares fitting algorithm described by Halir and Flusser, Proceeding of International Conference in Central Europe on Computer Graphics, Visualization and Interactive Digital Media: 125-132 (1998). The algorithm outputs the cell's major axis, which is the largest radius of the fitted ellipsis as described in the examples below.

In certain embodiments, the disclosure provides a method of predicting response to a hormone-directed therapy or chemotherapy in a prostate cancer (PCa) patient comprising (a) performing a direct analysis comprising immunofluorescent staining and morphological characteristization of nucleated cells in a blood sample obtained from the patient to identify and enumerate circulating tumor cells (CTC); (b) individually characterizing genotypic, morphometric and protein expression parameters to generate a profile for each of the CTCs; (c) repeating steps (a) and (b) at one or more timepoints after initial diagnosis of prostate cancer, and (d) predicting response to hormone-directed therapy in the prostate cancer patient based on sequentially monitoring of the profile at different timepoints after initial diagnosis of the prostate cancer. In particular examples, the timepoints are selected to correspond to clinical progression or therapy including, for example, systemic chemotherapy, hormone-directed therapy or radiation. In some examples, the blood samples (draws) are taken at timepoints at intervals representing decision points in the standard care of CRPC. For example, in addition to a sample taken at the time of initial diagnosis, the timepoints can be prior to initiation of docetaxel based chemotherapy, prior to initiation of a second line hormone therapy, for example, abiraterone acetate or another highly-selective androgen synthesis inhibitor, as well as at intervals after initiation of second line therapy, for example, after 2, 3, 4, 5 ,5, 7, 8, 9, 10, 11, 12, 13, 14, 15 ,16, 17, 18, 19, 20, 21, 22, 23, 24, 25, 26, or more weeks of continuous abiraterone treatment. In some examples, a blood draw is taken during ongoing/continous treatment if a change in clinical symptoms is detected.

In certain embodiments, the method of predicting response to a hormone-directed therapy or chemotherapy in a prostate cancer (PCa) patient encompasses a comparison of the CTC profiles between the timepoints. In some embodiments, the predicted response is emergence of resistant disease. In certain examples, a predicted response of emergence of resistant disease is based on identification of a resistant CTC in a blood draw taken at any timepoint. In further embodiments, the resistant CTC is assigned to a clonal lineage that predominates resistant disease.

In some embodiments, re-emergence of AR positive CTCs that had been depleted at a prior timepoint during the course of the disease predicts emergence of resistant disease. In some embodiments, the re-emergence of the AR positive CTCs is accompanied by genomic alterations that were not dominant in CTCs extracted a prior timepoint. In further examples, the genomic alterations comprise AR or MYC amplification. In additional embodiments, the re-emergence of the AR positive cells is further accompanied by a morphometric change, in particular, a decrease in cell roundness.

The ability to identify the presence, emergence or re-emergence of a CTC that is representative of a resistant clonal population prior to clinical escape and emergence of resistant disease underlies the predicitive power of the claimed methods with with regard to response to a hormone-directed therapy or chemotherapy in a prostate cancer (PCa) patient. The ability to predict response to a hormone-directed therapy in a prostate cancer (PCa) patient can inform treatment decisions during a critical period preceeding clinical escape and provide a clinician with actionable information as to what treatment course to follow. In some examples, a determination of whether a resistant CTC is AR independent, AR ligand independent or both can inform subsequent treatment decisions, for example, if the resistant cell is AR positive, the patient is a candidate for AR targeted treatment despite being AR ligand independent.

In its broadest sense, a biological sample can be any sample that contains CTCs. A sample can comprise a bodily fluid such as blood; the soluble fraction of a cell preparation, or an aliquot of media in which cells were grown; a chromosome, an organelle, or membrane isolated or extracted from a cell; genomic DNA, RNA, or cDNA in solution or bound to a substrate; a cell; a tissue; a tissue print; a fingerprint; cells; skin, and the like. A biological sample obtained from a subject can be any sample that contains cells and encompasses any material in which CTCs can be detected. A sample can be, for example, whole blood, plasma, saliva or other bodily fluid or tissue that contains cells.

In particular embodiments, the biological sample is a blood sample. As described herein, a sample can be whole blood, more preferably peripheral blood or a peripheral blood cell fraction. As will be appreciated by those skilled in the art, a blood sample can include any fraction or component of blood, without limitation, T-cells, monocytes, neutrophiles, erythrocytes, platelets and microvesicles such as exosomes and exosome-like vesicles. In the context of this disclosure, blood cells included in a blood sample encompass any nucleated cells and are not limited to components of whole blood. As such, blood cells include, for example, both white blood cells (WBCs) as well as rare cells, including CTCs.

The samples of this disclosure can each contain a plurality of cell populations and cell subpopulation that are distinguishable by methods well known in the art *(e.g.,* FACS, immunohistochemistry). For example, a blood sample can contain populations of non-nucleated cells, such as erythrocytes (*e.g.,* 4-5 million/μl) or platelets (150,000-400,000 cells/μl), and populations of nucleated cells such as WBCs *(e.g.,* 4,500 - 10,000 cells/μl), CECs or CTCs (circulating tumor cells; *e.g.,* 2-800 cells/). WBCs may contain cellular subpopulations of, *e.g.,* neutrophils (2,500-8,000 cells/μl), lymphocytes (1,000-4,000 cells/μl), monocytes (100-700 cells/μl), eosinophils (50-500 cells/μl), basophils (25 - 100 cells/μl) and the like. The samples of this disclosure are non-enriched samples, *i.e.,* they are not enriched for any specific population or subpopulation of nucleated cells. For example, non-enriched blood samples are not enriched for CTCs, WBC, B-cells, T-cells, NK-cells, monocytes, or the like.

In some examples the sample is a blood sample obtained from a healthy subject or a subject deemed to be at high risk for protate cancer or metastasis of existing prostate cancer based on art known clinically established criteria including, for example, age, race, family snd history. In some examples the blood sample is from a subject who has been diagnosed with prostate cancer and/or mCRPC based on tissue or liquid biopsy and/or surgery or clinical grounds. In some examples, the blood sample is obtained from a subject showing a clinical manifestation of prostate cancer and/or mCRPC well known in the art or who presents with any of the known risk factors for prostate cancer and/or mCRPC.

In some examples, the methods of predicting response to a hormone-directed therapy or chemotherapy in a prostate cancer (PCa) patient can further encompass individual patient risk factors and imaging data, which includes any form of imaging modality known and used in the art, for example and without limitation, by X-ray computed tomography (CT), ultrasound, positron emission tomography (PET), electrical impedance tomography and magnetic resonance (MRI). It is understood that one skilled in the art can select an imaging modality based on a variety of art known criteria. As described herein, the methods of the invention can encompass one or more pieces of imaging data. In the methods disclosed herein, one or more individual risk factors can be selected from the group consisting of age, race, family history. It is understood that one skilled in the art can select additional individual risk factors based on a variety of art known criteria. As described herein, the methods of the invention can encompass one or more individual risk factors. Accordingly, parameters can include imaging data, individual risk factors and CTC data. As described herein, parameters also can include, but are not limited to, biological molecules comprising nucleotides, nucleic acids, nucleosides, amino acids, sugars, fatty acids, steroids, metabolites, peptides, polypeptides, proteins, carbohydrates, lipids, hormones, antibodies, regions of interest that serve as surrogates for biological macromolecules and combinations thereof *(e.g.,* glycoproteins, ribonucleoproteins, lipoproteins) as well as portions or fragments of a biological molecule.

CTC data can include both morphological features and immunofluorescent features. As will be understood by those skilled in the art, additional parameters can be biomarker that can include a biological molecule, or a fragment of a biological molecule, the change and/or the detection of which can be correlated, individually or combined with other measurable features, with prostate cancer and/or mCRPC. CTCs, which can be present a single cells or in clusters of CTCs, are often epithelial cells shed from solid tumors and are present in very low concentrations in the circulation of subjects. Accordingly, detection of CTCs in a blood sample can be referred to as rare event detection. CTCs have an abundance of less than 1:1,000 in a blood cell population, e.g., an abundance of less than 1:5,000, 1:10,000, 1:30,000, 1:50:000, 1:100,000, 1:300,000, 1:500,000, or 1:1,000,000. In some examples, the a CTC has an abundance of 1:50:000 to 1:100,000 in the cell population.

The samples of this disclosure may be obtained by any means, including, e.g., by means of solid tissue biopsy or fluid biopsy *(see, e.g.,* Marrinucci D. et al., 2012, Phys. Biol. 9 016003). Briefly, in particular examples, the process can encompass lysis and removal of the red blood cells in a 7.5 mL blood sample, deposition of the remaining nucleated cells on specialized microscope slides, each of which accommodates the equivalent of roughly 0.5 mL of whole blood. A blood sample may be extracted from any source known to include blood cells or components thereof, such as venous, arterial, peripheral, tissue, cord, and the like. The samples may be processed using well known and routine clinical methods (e.g., procedures for drawing and processing whole blood). In some examples, a blood sample is drawn into anti-coagulent blood collection tubes (BCT), which may contain EDTA or Streck Cell-Free DNA™. In other examples, a blood sample is drawn into CellSave® tubes (Veridex). A blood sample may further be stored for up to 12 hours, 24 hours, 36 hours, 48 hours, or 60 hours before further processing.

In some examples, the methods of this disclosure comprise an intitial step of obtaining a white blood cell (WBC) count for the blood sample. In certain examples, the WBC count may be obtained by using a HemoCue® WBC device (Hemocue, Ängelholm, Sweden). In some examples, the WBC count is used to determine the amount of blood required to plate a consistent loading volume of nucleated cells per slide and to calculate back the equivalent of CTCs per blood volume.

In some examples, the methods of this disclosure comprise an initial step of lysing erythrocytes in the blood sample. In some examples, the erythrocytes are lysed, *e.g.,* by adding an ammonium chloride solution to the blood sample. In certain examples, a blood sample is subjected to centrifugation following erythrocyte lysis and nucleated cells are resuspended, *e.g.*, in a PBS solution.

In some examples, nucleated cells from a sample, such as a blood sample, are deposited as a monolayer on a planar support. The planar support may be of any material, *e.g.,* any fluorescently clear material, any material conducive to cell attachment, any material conducive to the easy removal of cell debris, any material having a thickness of < 100 μm. In some examples, the material is a film. In some examples the material is a glass slide. In certain examples, the method encompasses an initial step of depositing nucleated cells from the blood sample as a monolayer on a glass slide. The glass slide can be coated to allow maximal retention of live cells *(See, e.g.,* Marrinucci D. et al., 2012, Phys. Biol. 9: 016003). In some examples, about 0.5 million, 1 million, 1.5 million, 2 million, 2.5 million, 3 million, 3.5 million, 4 million, 4.5 million, or 5 million nucleated cells are deposited onto the glass slide. In some examples, the methods of this disclosure comprise depositing about 3 million cells onto a glass slide. In additional examples, the methods of this disclosure comprise depositing between about 2 million and about 3 million cells onto the glass slide. In some examples, the glass slide and immobilized cellular samples are available for further processing or experimentation after the methods of this disclosure have been completed.

In some embodiments, the methods of this disclosure comprise an initial step of identifying nucleated cells in the non-enriched blood sample. In some embodiments, the nucleated cells are identified with a fluorescent stain. In certain embodiments, the fluorescent stain comprises a nucleic acid specific stain. In certain examples, the fluorescent stain is diamidino-2-phenylindole (DAPI). In some examples, immunofluorescent staining of nucleated cells comprises pan cytokeratin (CK), cluster of differentiation (CD) 45 and DAPI. In some embodiments, the immunofluorescent staining of nucleated cells comprises pan cytokeratin, cluster of differentiation (CD) 45, diamidino-2-phenylindole (DAPI) and androgen receptor (AR). In some embodiments further described herein, CTCs comprise distinct immunofluorescent staining from surrounding nucleated cells. In some embodiments, the distinct immunofluorescent staining of CTCs comprises DAPI (+), CK (+) and CD 45 (-). In some examples, the identification of CTCs further comprises comparing the intensity of pan cytokeratin fluorescent staining to surrounding nucleated cells. In some examples, the CTC data is generated by fluorescent scanning microscopy to detect immunofluorescent staining of nucleated cells in a blood sample. Marrinucci D. et al., 2012, Phys. Biol. 9 016003).

In particular examples, all nucleated cells are retained and immunofluorescently stained with monoclonal antibodies targeting cytokeratin (CK), an intermediate filament found exclusively in epithelial cells, a pan leukocyte specific antibody targeting the common leukocyte antigen CD45, and a nuclear stain, DAPI. The nucleated blood cells can be imaged in multiple fluorescent channels to produce high quality and high resolution digital images that retain fine cytologic details of nuclear contour and cytoplasmic distribution. While the surrounding WBCs can be identified with the pan leukocyte specific antibody targeting CD45, CTCs can be identified as DAPI (+), CK (+) and CD 45 (-). In the methods described herein, the CTCs comprise distinct immunofluorescent staining from surrounding nucleated cells.

In further examples, the CTC are high definition CTCs (HD-CTCs). HD-CTCs are CK positive, CD45 negative, contain an intact DAPI positive nucleus without identifiable apoptotic changes or a disrupted appearance, and are morphologically distinct from surrounding white blood cells (WBCs). DAPI (+), CK (+) and CD45 (-) intensities can be categorized as measurable features during HD-CTC enumeration as previously described (Figure 1). Nieva et al., Phys Biol 9:016004 (2012). The enrichment-free, direct analysis employed by the methods disclosed herein results in high sensitivity and high specificity, while adding high definition cytomorphology to enable detailed morphologic characterization of a CTC population known to be heterogeneous.

While CTCs can be identified as comprises DAPI (+), CK (+) and CD 45 (-) cells, the methods of the invention can be practiced with any other parameters that one of skill in the art selects for generating CTC data and/or identifying CTCs and CTC clusters. One skilled in the art knows how to select a morphological feature, biological molecule, or a fragment of a biological molecule, the change and/or the detection of which can be correlated with a CTC. Molecule parameters include, but are not limited to, biological molecules comprising nucleotides, nucleic acids, nucleosides, amino acids, sugars, fatty acids, steroids, metabolites, peptides, polypeptides, proteins, carbohydrates, lipids, hormones, antibodies, regions of interest that serve as surrogates for biological macromolecules and combinations thereof (e.g., glycoproteins, ribonucleoproteins, lipoproteins). The term also encompasses portions or fragments of a biological molecule, for example, peptide fragment of a protein or polypeptide

In some examples, the disclosed method of predicting response to a hormone-directed therapy in a prostate cancer (PCa) patient, which include a step of isolation of the CTCs from the sample, further comprise relocation from the initial fluorescent image acquisition and subsequent re-imaging of the CTCs followed by physical extraction of the CTCs. Included in some examples of the claimed methods is a method for HD-CTC fluid phase capture that can be divided into three discrete sequential steps: (1) CTC relocation, (2) cell extraction and (3) physical isolation and manipulation of single CTCs for downstream molecular analyses, as described in the examples provided herewith.

A person skilled in the art will appreciate that a number of methods can be used to generate CTC data, including microscopy based approaches, including fluorescence scanning microscopy *(see, e.g.,* Marrinucci D. et al., 2012, Phys. Biol. 9:016003), mass spectrometry approaches, such as MS/MS, LC-MS/MS, multiple reaction monitoring (MRM) or SRM and product-ion monitoring (PIM) and also including antibody based methods such as immunofluorescence, immunohistochemistry, immunoassays such as Western blots, enzyme-linked immunosorbant assay (ELISA), immunopercipitation, radioimmunoassay, dot blotting, and FACS. Immunoassay techniques and protocols are generally known to those skilled in the art (Price and Newman, Principles and Practice of Immunoassay, 2nd Edition, Grove's Dictionaries, 1997; and Gosling, Immunoassays: A Practical Approach, Oxford University Press, 2000.) A variety of immunoassay techniques, including competitive and non-competitive immunoassays, can be used (Self et al., Curr. Opin. Biotechnol., 7:60-65 (1996), *see also* John R. Crowther, The ELISA Guidebook, 1st ed., Humana Press 2000, ISBN 0896037282 and, An Introduction to Radioimmunoassay and Related Techniques, by Chard T, ed., Elsevier Science 1995, ISBN 0444821198).

A person of skill in the art will futher appreciate that the presence or absence of parameters may be detected using any class of marker-specific binding reagents known in the art, including, e.g., antibodies, aptamers, fusion proteins, such as fusion proteins including protein receptor or protein ligand components, or parameter-specific small molecule binders. In some examples, the presence or absence of CK or CD45 is determined by an antibody.

The antibodies of this disclosure bind specifically to a parameter. The antibody can be prepared using any suitable methods known in the art. *See, e.g.,* Coligan, Current Protocols in Immunology (1991); Harlow & Lane, Antibodies: A Laboratory Manual (1988); Goding, Monoclonal Antibodies: Principles and Practice (2d ed. 1986). The antibody can be any immunoglobulin or derivative thereof, whether natural or wholly or partially synthetically produced. All derivatives thereof which maintain specific binding ability are also included in the term. The antibody has a binding domain that is homologous or largely homologous to an immunoglobulin binding domain and can be derived from natural sources, or partly or wholly synthetically produced. The antibody can be a monoclonal or polyclonal antibody. In some examples, an antibody is a single chain antibody. Those of ordinary skill in the art will appreciate that antibody can be provided in any of a variety of forms including, for example, humanized, partially humanized, chimeric, chimeric humanized, *etc.* The antibody can be an antibody fragment including, but not limited to, Fab, Fab', F(ab')2, scFv, Fv, dsFv diabody, and Fd fragments. The antibody can be produced by any means. For example, the antibody can be enzymatically or chemically produced by fragmentation of an intact antibody and/or it can be recombinantly produced from a gene encoding the partial antibody sequence. The antibody can comprise a single chain antibody fragment. Alternatively or additionally, the antibody can comprise multiple chains which are linked together, for example, by disulfide linkages, and any functional fragments obtained from such molecules, wherein such fragments retain specific-binding properties of the parent antibody molecule. Because of their smaller size as functional components of the whole molecule, antibody fragments can offer advantages over intact antibodies for use in certain immunochemical techniques and experimental applications.

A detectable label can be used in the methods described herein for direct or indirect detection of the parameters when generating CTC data in the methods of the invention. A wide variety of detectable labels can be used, with the choice of label depending on the sensitivity required, ease of conjugation with the antibody, stability requirements, and available instrumentation and disposal provisions. Those skilled in the art are familiar with selection of a suitable detectable label based on the assay detection of the parameters in the methods of the invention. Suitable detectable labels include, but are not limited to, fluorescent dyes (*e.g.*, fluorescein, fluorescein isothiocyanate (FITC), Oregon Green™, rhodamine, Texas red, tetrarhodimine isothiocynate (TRITC), Cy3, Cy5, Alexa Fluor® 647, Alexa Fluor® 555, Alexa Fluor® 488), fluorescent markers (*e.g.*, green fluorescent protein (GFP), phycoerythrin, etc.), enzymes (*e.g.*, luciferase, horseradish peroxidase, alkaline phosphatase, etc.), nanoparticles, biotin, digoxigenin, metals, and the like.

For mass-sectrometry based analysis, differential tagging with isotopic reagents, e.g., isotope-coded affinity tags (ICAT) or the more recent variation that uses isobaric tagging reagents, iTRAQ (Applied Biosystems, Foster City, Calif.), followed by multidimensional liquid chromatography (LC) and tandem mass spectrometry (MS/MS) analysis can provide a further methodology in practicing the methods of this disclosure.

A chemiluminescence assay using a chemiluminescent antibody can be used for sensitive, non-radioactive detection of proteins. An antibody labeled with fluorochrome also can be suitable. Examples of fluorochromes include, without limitation, DAPI, fluorescein, Hoechst 33258, R-phycocyanin, β-phycoerythrin, R-phycoerythrin, rhodamine, Texas red, and lissamine. Indirect labels include various enzymes well known in the art, such as horseradish peroxidase (HRP), alkaline phosphatase (AP), beta-galactosidase, urease, and the like.

Detection systems using suitable substrates for horseradish-peroxidase, alkaline phosphatase, beta.-galactosidase are well known in the art.

A signal from the direct or indirect label can be analyzed, for example, using a microscope, such as a fluorescence microscope or a fluorescence scanning microscope. Alternatively, a spectrophotometer can be used to detect color from a chromogenic substrate; a radiation counter to detect radiation such as a gamma counter for detection of $^{125}I$; or a fluorometer to detect fluorescence in the presence of light of a certain wavelength. If desired, assays used to practice the methods of this disclosure can be automated or performed robotically, and the signal from multiple samples can be detected simultaneously.

In some embodiments, the parameters are immunofluorescent markers. In some embodiments, the immunofluorescent makers comprise a marker specific for epithelial cells In some embodiments, the immunofluorescent makers comprise a marker specific for white blood cells (WBCs). In some embodiments, one or more of the immunofluorescent markers comprise CD45 and CK.

In some embodiments, the presence or absence of immunofluorescent markers in nucleated cells, such as CTCs or WBCs, results in distinct immunofluorescent staining patterns. Immunofluorescent staining patterns for CTCs and WBCs may differ based on which epithelial or WBC markers are detected in the respective cells. In some embodiments, determining presence or absence of one or more immunofluorescent markers comprises comparing the distinct immunofluorescent staining of CTCs with the distinct immunofluorescent staining of WBCs using, for example, immunofluorescent staining of CD45, which distinctly identifies WBCs. There are other detectable markers or combinations of detectable markers that bind to the various subpopulations of WBCs. These may be used in various combinations, including in combination with or as an alternative to immunofluorescent staining of CD45.

In some embodiments, CTCs comprise distinct morphological characteristics compared to surrounding nucleated cells. In some embodiments, the morphological characteristics comprise nucleus size, nucleus shape, cell size, cell shape, and/or nuclear to cytoplasmic ratio. In some examples, the method further comprises analyzing the nucleated cells by nuclear detail, nuclear contour, presence or absence of nucleoli, quality of cytoplasm, quantity of cytoplasm, intensity of immunofluorescent staining patterns. A person of ordinary skill in the art understands that the morphological characteristics of this disclosure may include any feature, property, characteristic, or aspect of a cell that can be determined and correlated with the detection of a CTC.

CTC data can be generated with any microscopic method known in the art. In some examples, the method is performed by fluorescent scanning microscopy. In certain examples the microscopic method provides high-resolution images of CTCs and their surrounding WBCs (see, e.g., Marrinucci D. et al., 2012, Phys. Biol. 9:016003)). In some examples, a slide coated with a monolayer of nucleated cells from a sample, such as a non-enriched blood sample, is scanned by a fluorescent scanning microscope and the fluorescence intensities from immunofluorescent markers and nuclear stains are recorded to allow for the determination of the presence or absence of each immunofluorescent marker and the assessment of the morphology of the nucleated cells. In some examples, microscopic data collection and analysis is conducted in an automated manner.

In some examples, a CTC data includes detecting one or more parameters, for example, CK and CD 45. A parameter is considered "present" in a cell if it is detectable above the background noise of the respective detection method used (e.g., 2-fold, 3-fold, 5-fold, or 10-fold higher than the background; e.g., $2\sigma$ or $3\sigma$ over background). In some examples, a parameter is considered "absent" if it is not detectable above the background noise of the detection method used (e.g., <1.5-fold or <2.0-fold higher than the background signal; e.g., $<1.5\sigma$ or $<2.0\sigma$ over background).

In some examples, the presence or absence of immunofluorescent markers in nucleated cells is determined by selecting the exposure times during the fluorescence scanning process such that all immunofluorescent markers achieve a pre-set level of fluorescence on the WBCs in the field of view. Under these conditions, CTC-specific immunofluorescent markers, even though absent on WBCs are visible in the WBCs as background signals with fixed heights. Moreover, WBC-specific immunofluorescent markers that are absent on CTCs are visible in the CTCs as background signals with fixed heights. A cell is considered positive for an immunofluorescent marker (i.e., the marker is considered present) if its fluorescent signal for the respective marker is significantly higher than the fixed background signal (e.g., 2-fold, 3-fold, 5-fold, or 10-fold higher than the background; e.g., $2\sigma$ or $3\sigma$ over background). For example, a nucleated cell is considered CD 45 positive (CD 45$^+$) if its fluorescent signal for CD 45 is significantly higher than the background signal. A cell is considered negative for an immunofluorescent marker (i.e., the marker is considered absent) if the cell's fluorescence signal for the respective marker is not significantly above the background signal (e.g., <1.5-fold or <2.0-fold higher than the background signal; e.g., $< 1.5\sigma$ or $<2.0\sigma$ over background).

Typically, each microscopic field contains both CTCs and WBCs. In certain examples, the microscopic field shows at least 1, 5, 10, 20, 50, or 100 CTCs. In certain examples, the microscopic field shows at least 10, 25, 50, 100, 250, 500, or 1,000 fold more WBCs than CTCs. In certain examples, the microscopic field comprises one or more CTCs or CTC clusters surrounded by at least 10, 50, 100, 150, 200, 250, 500, 1,000 or more WBCs.

In some examples of the methods described herein, generation of the CTC data comprises enumeration of CTCs that are present in the blood sample. In some examples, the methods described herein encompass detection of at least 1.0 CTC/mL of blood, 1.5 CTCs/mL of blood, 2.0 CTCs/mL of blood, 2.5 CTCs/mL of blood, 3.0 CTCs/mL of blood, 3.5 CTCs/mL of blood, 4.0 CTCs/mL of blood, 4.5 CTCs/mL of blood, 5.0 CTCs/mL of blood, 5.5 CTCs/mL of blood, 6.0 CTCs/mL of blood, 6.5 CTCs/mL of blood, 7.0 CTCs/mL of blood, 7.5 CTCs/mL of blood, 8.0 CTCs/mL of blood, 8.5 CTCs/mL of blood, 9.0 CTCs/mL of blood, 9.5 CTCs/mL of blood, 10 CTCs/mL of blood, or more.

In some examples of methods described herein, generation of the CTC data comprises detecting distinct subtypes of CTCs, including non-traditional CTCs. In some examples, the methods described herein encompass detection of at least 0.1 CTC cluster/mL of blood, 0.2 CTC clusters/mL of blood, 0.3 CTC clusters/mL of blood, 0.4 CTC clusters/mL of blood, 0.5 CTC clusters/mL of blood, 0.6 CTC clusters/mL of blood, 0.7 CTC clusters/mL of blood, 0.8 CTC clusters/mL of blood, 0.9 CTC clusters/mL of blood, 1 CTC cluster/mL of blood, 2 CTC clusters/mL of blood, 3 CTC clusters/mL of blood, 4 CTC clusters/mL of blood, 5 CTC clusters/mL of blood, 6 CTC clusters/mL of blood, 7 CTC clusters/mL of blood, 8 CTC clusters/mL of blood, 9 CTC clusters/mL of blood, 10 clusters/mL or more. In a particular example, the methods described herein encompass detection of at least 1 CTC cluster/mL of blood.

In some examples, the methods of predicting response to a hormone-directed therapy or chemotherapy in a prostate cancer (PCa) patient can further encompass the use of a predictive model. In further examples, the methods of predicting response to a hormone-directed therapy in a prostate cancer (PCa) patient can further encompass comparing a measurable feature with a reference feature. As those skilled in the art can appreciate, such comparison can be a direct comparison to the reference feature or an indirect comparison where the reference feature has been incorporated into the predictive model. In further examples, analyzing a measurable feature to prospectively identify resistance to hormone directed therapies in a PCa patient encompasses one or more of a linear discriminant analysis model, a support vector machine classification algorithm, a recursive feature elimination model, a prediction analysis of microarray model, a logistic regression model, a CART algorithm, a flex tree algorithm, a LART algorithm, a random forest algorithm, a MART algorithm, a machine learning algorithm, a penalized regression method, or a combination thereof. In particular examples, the analysis comprises logistic regression. In additional examples, the prediction of resistance to hormone directed therapies in a PCa patient is expressed as a risk score.

An analytic classification process can use any one of a variety of statistical analytic methods to manipulate the quantitative data and provide for classification of the sample. Examples of useful methods include linear discriminant analysis, recursive feature elimination, a prediction analysis of microarray, a logistic regression, a CART algorithm, a FlexTree algorithm, a LART algorithm, a random forest algorithm, a MART algorithm, machine learning algorithms and other methods known to those skilled in the art.

Classification can be made according to predictive modeling methods that set a threshold for determining the probability that a sample belongs to a given class. The probability preferably is at least 50%, or at least 60%, or at least 70%, or at least 80%, or at least 90% or higher. Classifications also can be made by determining whether a comparison between an obtained dataset and a reference dataset yields a statistically significant difference. If so, then the sample from which the dataset was obtained is classified as not belonging to the reference dataset class. Conversely, if such a comparison is not statistically significantly different from the reference dataset, then the sample from which the dataset was obtained is classified as belonging to the reference dataset class.

The predictive ability of a model can be evaluated according to its ability to provide a quality metric, e.g. AUROC (area under the ROC curve) or accuracy, of a particular value, or range of values. Area under the curve measures are useful for comparing the accuracy of a classifier across the complete data range. Classifiers with a greater AUC have a greater capacity to classify unknowns correctly between two groups of interest. ROC analysis can be used to select the optimal threshold under a variety of clinical circumstances, balancing the inherent tradeoffs that exist between specificity and sensitivity. In some examples, a desired quality threshold is a predictive model that will classify a sample with an accuracy of at least about 0.7, at least about 0.75, at least about 0.8, at least about 0.85, at least about 0.9, at least about 0.95, or higher. As an alternative measure, a desired quality threshold can refer to a predictive model that will classify a sample with an AUC of at least about 0.7, at least about 0.75, at least about 0.8, at least about 0.85, at least about 0.9, or higher.

As is known in the art, the relative sensitivity and specificity of a predictive model can be adjusted to favor either the specificity metric or the sensitivity metric, where the two metrics have an inverse relationship. The limits in a model as described above can be adjusted to provide a selected sensitivity or specificity level, depending on the particular requirements of the test being performed. One or both of sensitivity and specificity can be at least about 0.7, at least about 0.75, at least about 0.8, at least about 0.85, at least about 0.9, or higher.

The raw data can be initially analyzed by measuring the values for each measurable feature or parameter, usually in triplicate or in multiple triplicates. The data can be manipulated, for example, raw data can be transformed using standard curves, and the average of triplicate measurements used to calculate the average and standard deviation for each patient. These values can be transformed before being used in the models, *e.g.* log-transformed, Box-Cox transformed (Box and Cox, Royal Stat. Soc., Series B, 26:211-246(1964). The data are then input into a predictive model, which will classify the sample according to the state. The resulting information can be communicated to a patient or health care provider.

In some examples, the methods of predicting response to a hormone-directed therapy in a prostate cancer (PCa) patient can have a specificity of >60%, >70%, >80%, >90% or higher. In additional examples, the methods of predicting response to a hormone-directed therapy in a prostate cancer (PCa) patient can have a specificity >90% at a classification threshold of 7.5 CTCs/mL of blood.

As will be understood by those skilled in the art, an analytic classification process can use any one of a variety of statistical analytic methods to manipulate the quantitative data and provide for classification of the sample. Examples of useful methods include, without limitation, linear discriminant analysis, recursive feature elimination, a prediction analysis of microarray, a logistic regression, a CART algorithm, a FlexTree algorithm, a LART algorithm, a random forest algorithm, a MART algorithm, and machine learning algorithms.

The following examples are provided by way of illustration, not limitation.

## EXAMPLES

### Example 1. Rapid Phenotypic and Genomic Change in Response to Therapeutic Pressure in Prostate Cancer Detected by High Content Analysis of Single CTCs

This example shows monitoring of treatment response by longitudinal CTC molecular analysis and demonstrates that phenotypic and genotypic changes in circulating cell populations represent sequential steps of genetic evolution in response to a multi-step therapeutic regime culminating in treatment with abiraterone acetate

**Patient Clinical History and Blood Draws Collected During Treatment.** The study was approved by the institutional review board (IRB) of University of Southern California Comprehensive Cancer Center. The patient provided written informed consent. The patient presented with PCa metastatic to a lumbar vertebrae at diagnosis for which the primary biopsy represents the first specimen in this study. Initial treatment consisted of androgen deprivation therapy (leuprolide acetate). After 5 months, there was clinical progression to CRPC and the patient was enrolled in a clinical trial of docetaxel combined with bevacizumab and everolimus (clinicaltrials.gov identifier: NCT00574769). Before chemotherapy was initiated, a baseline blood draw was taken (Draw 1) according to the sample collection protocol. Clinical progression was noted after 4 months of protocol-specified chemotherapy. Over the next 3 months, additional doses of docetaxel as well as external-beam radiotherapy and samarium (153Sm) lexidronam (a bone-targeting radiopharmaceutical) were employed with limited palliative benefit. At 12 months after diagnosis, treatment with abiraterone acetate, a highly-selective androgen synthesis inhibitor, was initiated. Blood was drawn prior to starting abiraterone (Draw 2), at 3 weeks of continuous treatment coinciding with a clinical response represented by decreased pain and PSA level (Draw 3), and at 9 weeks coinciding with clinical progression represented by increasing pain and PSA levels (Draw 4). Following abiraterone, treatment was changed to cabazitaxel without clinical response followed by a rapid clinical deterioration. The patient died of widely metastatic prostate cancer 4 months following Draw 4 (17 months after diagnosis).

**Blood Sample Collection and Processing for CTC Detection.** Patient peripheral blood samples were collected according to an IRB approved protocol. Samples were shipped to our laboratory and processed within 24 hours after the time of draw. Sample preparation was previously described in Marrinucci et al., Phys Biol 9: 016003 (2012). In brief, it consists of a red blood cell lysis followed by plating of the nucleated cells as a monolayer on custom made cell-adhesion glass slide followed by storage in a biorepository. Each sample produced at least 14 independent slides for CTC identification and characterization.

**Immunofluorescence Staining and CTC Enumeration.** For this study, we used a protocol based on the published HD-CTC assay coupled with evaluation of androgen receptor (AR) status within the cytokeratin (CK) positive CTC population. Lazar et al., Phys Biol 9: 016002 (2012). Briefly, the cells were labeled using mouse monoclonal cytokeratin 19 (1:100; Dako) and panCK (1:100; Sigma) primary antibodies to identify cytokeratin (CK) positive cells. AR positive HD-CTCs were identified using a rabbit anti-AR monoclonal antibody (1:250, Cell Signaling Technology). Both the CK and AR antigens were visualized using AlexaFluor secondary antibodies; the CK primary antibodies were recognized with Alexa Fluor 555 IgG1 secondary antibody (1:500, Invitrogen) and the rabbit AR antibody was recognized with Alexa Fluor 488 IgG (H+L) secondary antibody (1:1000, Invitrogen). Alexa Fluor 647 conjugated anti-CD45 (1:125; AbD Serotec) primary antibody was used to identify leukocytes as an exclusion marker. To confirm that the cells are nucleated and to enable the analysis of nuclear morphology all cells were stained with a 4',6-diamidino-2-phenylindole (DAPI).

The slides were imaged and putative CTCs were recorded using a computerized high-throughput fluorescence microscope at $10\times$ magnification. CTCs were identified by a hematology technician using the previously published criteria of having a DAPI+ nucleus plus cytokeratin positivity and CD45 negativity. Marrinucci et al., Phys Biol 9: 016003 (2012). Androgen receptor protein expression and localization were evaluated using two criteria (1) presence (AR+) or absence (AR-) of AR staining, and (2) AR subcellular localization (nuclear AR versus cytoplasmic staining or both). The threshold for AR positivity was defined as a signal more than 6 standard deviations over the mean signal intensity (SDOM) observed in the surroundings leukocytes (background). Subcellular localization was measured using the relative pixel density of AR staining over the nucleus and cytoplasm.

**HD-CTC Assay Reproducibility.** The HD-CTC assay was technically validated with cell line spiking experiments to reach an R2 = 0.9997 on linearity testing as previously reported. These experiments were performed using SK-BR-3 cell lines and 0 to $3 \times 102$ cells per mL of normal donor control blood. The coefficient of variation is 16% and inter-processor correlation is R2 = 0.979. Sample preparation process adhered to standard operating procedures for patient samples through a bar coded system for all consumables and instrumentation. All off-the-shelf instrumentation was calibrated according to the technical validation protocols established during the commissioning. Nair et al., PLoS One 8: e67733 (2013)

**Extraction of Single Cells.** As a standard procedure, aimed at minimizing DNA fragmentation cells were picked within 5 days of the initial staining procedure. The experimental protocol for HD-CTC fluid phase capture was divided into three discrete sequential steps: (1) CTC relocation, (2) cell extraction and (3) isolation and manipulation of single CTCs for downstream molecular analyses.

HD-CTCs were relocated (step 1) using a transformation matrix from the initial data acquisition for HD-CTC identification. After calibration and relocation, each candidate cell was re-imaged at $40 \times$ resolution for the detailed morphometric analysis. For the cell extraction (step 2) an Eppendorf Transfer Man NK2 micromanipulator was used to capture the cell of interest inside a 25° jagged micropipette (Piezo Drill Tip ES, Eppendorf) by applying fluid suction. Once the cell of interest was captured inside the micropipette (step 3), the cell was rinsed with PBS and deposited inside a 0.2 mL PCR tube containing 2 $\mu$L of lysis buffer (200 mM KOH; 50 mM DTT). The sample was then and immediately frozen and stored at -80°C until further processing. All instruments and consumables were decontaminated using a DNAase solution and exposure to UV light for 30 min prior to the experiment.

**Single Cell Next Generation Sequencing and Bioinformatic Analysis.** The cell containing vials were transferred in dry ice to the sequencing laboratory. Briefly, the lysed cell mixture was thawed and subjected to WGA and sequencing library construction as previously reported by Baslan et al, Nat Protoc 7: 1024-1041 (2012).

WGA was carried out manually in a 96-well plate format using the WGA4 Genomeplex Single Cell Whole Genome Amplification Kit (Sigma-Aldrich), followed by purification using a QIAquick 96 PCR Purification Kit (Qiagen). Concentration of eluted DNA was measured using a Nanodrop 8000 (Thermo Scientific). For each well, amplification was considered successful if the resulting DNA concentration was ≥70 ng/ $\mu$l (elution volume of 50 $\mu$l), followed by further Quality Control (QC) to confirm the appropriate sample size distribution using the Agilent 2100 Bioanalyzer (High-Sensitivity DNA Assay and Kit, Agilent Technologies).

In addition, detailed methods used to analyze sequencing data were published recently by our group in Baslan et al, Nat Protoc 7: 1024-1041 (2012). Briefly, the informatics methods involves three steps: first, deconvoluting the sequence reads based on barcodes; second, mapping the reads to the human genome (hg19, Genome Reference Consortium GRCh37, UCSC Genome Browser database) (Meyer et al., Nucleic Acids Res 41: D64-69 (2013)), and removing PCR duplicates; and third, normalizing for guanine-cytosine (GC) content and estimating copy number using the CBS segmentation algorithm. The copy number profiles in this report are based on 20,000 variable length genome bins, averaging a length of -150 kilo-base pairs each, and were calculated as ratio compared to normal (hg 19). The data reported here had a median count of 1.78 million uniquely mapping reads, with a range from 244,190 (minimum cut off 200,000) to 5.33 million.

**Cluster Analysis.** The hierarchical clustering was performed in R (Team RC (2012) R: A Language and Environment for Statistical Computing) using the heatmap.2 function in the gplots package. Ward's method with Euclidean distance metric was used for the clustering. The heatmap is colored according to the cutoffs described above and the clustering was performed using median centered data.

**Frequency Analysis to Define Genomic Alterations.** Using median centered CNV profiles, cutoff ratios versus the median of 0.8 and 1.25 were used to define deletions and amplifications, respectively. These cutoffs were used both to color the heatmap and to do the frequency analysis.

**Statistics and Cell Morphology Analysis.** The cell shape (cell roundness) was analyzed by tracing the cell cytoplasm contour in the composite image of each CTC. The traced cell image was imported into R, and an ellipsis was fitted to the shape using a least squares fitting algorithm described by Halir and Flusser, Proceeding of International Conference in Central Europe on Computer Graphics, Visualization and Interactive Digital Media: 125-132 (1998). The algorithm outputs the cell's major axis, which is the largest radius of the fitted ellipse (Refer to supporting information). The cell roundness (c) is estimated as the fraction of the de facto cell area (A) and the area of a circle with the radius (r) set to the cell's major axis.

$$C = A / \pi r^2$$

The p-value used in the comparison of the roundness between the CTCs in Draw 3 and 4 was calculated using the Wilcoxon sum-rank test.

In order to assess the patient's response to treatment high content single cell analysis including: (1) AR protein expression phenotype, (2) AR subcellular localization and (3) CNV genomic profiling were performed in the CTCs identified in the blood samples collected across four different intervals representing decision points in the standard care of CRPC including: (Draw 1) immediately prior to initiation of docetaxel based chemotherapy, (Draw 2) immediately prior to abiraterone acetate (a highly-selective androgen synthesis inhibitor), (Draw 3) after three weeks, and (Draw 4) after nine weeks of continuous abiraterone treatment. The specific data for all profiled cells is presented in the supporting information. In addition, a similar sequencing based method was used to obtain the CNV profile of one metastatic site from the patient using a bone biopsy taken at the time of diagnosis (5 months prior to draw 1) prior to receiving any cancer-specific therapy. As shown in Figure 1A, and during the 7 month period between Draws 1 and 2, the patient exhibited initial response to docetaxel-based chemotherapy followed by resistance. Concurrently, the patient's fluid biopsy showed a constant proportion of AR+ and AR- subpopulations while the overall number of CTCs declined (Figure 1A, ID, Figure 5 and Figure 8).

The genomic CNV profiles of CK+ cells from Draws 1 and 2 were of two types (Figures 2 and 6). Three of these cells were negative for AR expression (CK+AR-) while the majority (16/19) showed high levels of AR protein (CK+AR+). One AR- and one AR+ cell had near normal CNV profiles comparable to those obtained from single CK-CD45+ leukocytes (Figure 2). All other CK+AR+ cells exhibited a complex pattern of genomic rearrangements that were similar to the genomic profile obtained retrospectively from the patient's bone metastasis (hormone naive tissue sample) obtained at diagnosis (Figure 2 and Figure 3A). The CK+AR+ cells and the bone metastasis sample shared multiple gains and losses of chromosome arms plus a characteristic focal amplification on 3p13 centered on the phosphatase regulatory subunit PPP4R2 and containing at least two genes implicated in cancer, FoxP1 (Taylor et al., Cancer Cell 18: 11-22 (2010); Goatly et al., Mod Pathol 21: 902-911 (2008)) and MITF (Garraway et al., Nature 436: 117-122 (2005)) (Figure 2). To the level of resolution available, each of the shared events showed identical genomic breakpoints, and in the hierarchical clustering analysis the AR+ cells from draws 1 and 2 clustered together with the bone metastasis (Cluster A in Figure 3A). From this evidence, we infer that these cells are bona fide CTCs derived from the patient's metastatic lineage. Despite the clear lineage relationship, the AR+ circulating cells differed from the metastasis at the AR locus, showing multicopy amplification of various segments on Xq12 containing the AR gene itself. AR amplification is frequent in CRPC, and has been linked to progression from castration-sensitive prostate cancer to CRPC. Koivisto et al., Cancer Res 57: 314-319 (1997). It is noteworthy that each of the AR amplifications (Figure 3C) are unique, arising from multiple different breakpoints on either side of the AR gene, indicating that AR amplification arose multiple independent times (convergent evolution) likely as result of the selective pressure imposed by the androgen deprivation therapy.

At Draw 3, after three weeks of abiraterone acetate treatment, the patient displayed a clear clinical response as defined by decrease in PSA and pain (Figure 1B). This response coincided with an abrupt change in CTC phenotypes and genotypes. Although the absolute number of CTCs in Draw 3 was comparable to that of Draw 2, there was an almost complete depletion of the AR+ CTC population (Figure 1A). The CK+ cells identified in Draw 3 expressed little or no AR protein and also differed morphologically, appearing to be significantly more elongated than the AR+ cells from Draws 1 and 2 (Figure 5 and Figure 8). This morphological change is reflected in a decrease in the median cell roundness (Figure 7) from 0.87 (sd = 0.14) in Draw 1 and 2 to 0.62 (sd = 0.15) in Draw 3, $p<10^{-11}$ Wilcoxon rank-sum test (Figure 1C).

The apparent effect of treatment was also evident in the genomic analysis of Draw 3 where the altered phenotypic states correlated with distinct genomic profiles. The majority (10/12) of phenotypically AR- cells from Draw 3 were not amplified for AR and exhibited apparently normal or near normal (pseudodiploid) profiles (Figure 6) placing them in Cluster B in Figures 3A. One of the two AR- cells from this timepoint had the CNV signature typical of Cluster A including amplification of AR, while the other associated with a third cluster (Cluster C in Figure 3A), dominated by cells from the subsequent timepoint (Draw 4). Missense mutations affecting AR protein stability and/or nonsense mutations in the AR gene could account for the AR phenotype-genotype disparity in the last two cells. We interpret that the initial response to abiraterone acetate significantly depleted the androgen-dependent AR+ population, and that another AR- population dominated by pseudodiploid cells was present in the circulation. Based on the total cell count, staying constant between draws 2 and 3, we infer that the Draw 3 population is a consequence of cancer, but from a source outside of the main tumor lineage (Figure 3A).

Draw 4 was collected at the point of clinical progression, when PSA levels increased after 9 weeks on abiraterone (Figure 1B). At this point, the CTC count had decreased to 47% of the previous timepoint, but had once again undergone a significant phenotypic shift, as the majority of CTCs were once again AR+ with a cell roundness value of 0.81 typical of cells from the first two draws (Figure 1C and Figure 5). This finding, suggesting an association between therapy response and a CTC phenotype rather than with total CTC count, is consistent with a recently published study where the expression of two markers for the AR signaling pathway on CTCs was monitored in response to androgen-directed therapy. Miyamoto et al., Cancer Discov 2: 995-1003 (2012).

Alterations in response to therapy were again apparent at the genomic level, as (6/10) cells formed the majority of a new, apparently clonal, subpopulation (Cluster C in Figures 3A and S2). The CNV signatures in Cluster C are clearly in the original lineage, going back to the bone metastasis sampled before any systemic therapy, but is now characterized by functionally relevant events such as a narrow amplicon containing MYC, and the disappearance of the FOXP1/MITF amplicon along with other differences noted in Figures 2, 3A and 3B. MYC amplification is one of the most common alterations observed in metastatic tumors, and has been suggested to be a bypass mechanism for AR independent resistance. Koh et al., Genes Cancer 1: 617-628 (2010). Interestingly a closer examination of the genomic AR amplification (outlined in Figure 3C) shows that, in contrast to the heterogeneous amplification boundaries observed in earlier cells (cluster A), the cells in cluster C exhibit a single profile shape with nearly uniform breakpoints and significantly higher levels of AR amplification. Taken together the genomic elements suggest that the Cluster C cells represent a novel lineage, apparently resistant to abiraterone acetate, and generated perhaps from a single resistant cell.

In addition, morphometric analysis of AR subcellular localization showed that AR was generally localized in the nucleus of cells from Draws 1 and 2, but was identified as significantly less localized to the nucleus in the CTCs isolated in Draw 4 collected at progression (p = 0.00017 Wilcoxon rank-sum test) (Figure 4). This finding is particularly interesting in the light of recent studies indicating that ligand independent AR splice variants may mediate abiraterone resistance in a human CRPC xenograft model (Mostaghel et al., Clin Cancer Res 17: 5913-5925(2011)), and that these truncated and constitutively active forms of AR is found to be localized in the nucleus as well as cytoplasm in prostate cancer cell lines. Chan et al., J Biol Chem 287: 19736-19749(2012).

Although our study is based on longitudinal study of a single patient, our findings are consistent with previous studies involving genomic analysis from either CTCs or circulating cell-free DNA isolated from patients with metastatic prostate cancer. Magbanua et al., BMC Cancer 12: 78 (2012), Heitzer et al., Genome Med 5: 30 (2013). However, these prior studies were generally limited to the characterization of pooled samples from a single timepoint, and therefore do not shed light into the temporal and dynamic evolution of cancer under therapeutic selective pressure. Regardless, consistent with these prior reports we observed copy number alterations in chromosome 8 (particularly gain in 8q and loss in 8p), which is one of the most frequent somatic mutations described in prostate cancer. Taylor et al., Cancer Cell 18: 11-22 (2010). In addition, our finding that AR amplification was not found in sample obtained before initial androgen-deprivation therapy, but occurred at high frequency in later samples representing CRPC is consistent with multiple prior studies linking AR amplification with androgen-independent prostate cancer growth.

Clonal evolution of cancer is a well-established principle that has been validated in multiple published studies (Navin et al., Genome Res 20: 68-80 (2010), Gerlinger et al., N Engl J Med 366: 883-892 (2012), Almendro et al., Cancer Res 74: 1338-1348 (2014)), as well as, the appearance of somatic mutations in tumors in response to therapeutic selective pressure Sequist et al., Sci Transl Med 3 (2011), Shi et al. Cancer Discov 4: 80-93 (2014). We interpret the phenotypic and genotypic changes in circulating cell populations presented here as representing sequential steps of genetic evolution in response to a multi-step therapeutic regime culminating in treatment with abiraterone acetate.

The bulk metastatic biopsy taken prior to initiation of therapy provides the root CNV profile to from which the subsequent time course CTC profiles have evolved. It exhibits a backbone of CNV elements that defines a lineage, based on CNV breakpoints, that is carried forward in the circulating cells from blood draws taken during later treatment. The first two of these draws were taken after an initial course of androgen deprivation therapy (ADT) (leuprolide acetate). One population in Draws 1 and 2 (Clone A) was a clearly a direct descendant of the met biopsy profile with the exception that all cells showed high-copy AR amplification and strong AR protein expression. We interpret these cells to be the products of metastatic deposits that had evolved to amplify the AR gene locus and overexpress androgen receptor protein as a result of genetic selection for resistance to the initial round of ADT. It is noteworthy that in addition to the AR amplified cells, both draws contained a significant fraction of cytokeratin positive, AR negative cells with near-normal (pseudodiploid) genomes forming a separate CNV cluster (Figure 3). It is also interesting that the clonal structure of the AR+ cells changed very little between Draws 1 and 2 despite intervening rounds of chemotherapy and radiation therapy over a period of 7 months.

In contrast to the similarity of cell phenotype and genotype in Draws 1 and 2, the selective effects of abiraterone acetate were very evident in Draws 3 and 4. After three weeks of treatment the androgen dependent, AR positive cells in Draws 1 and 2 were nearly absent and the CK+ population consisted almost entirely of AR negative pseudodiploid cells. The clone (Clone C) that would become dominant at the nine-week timepoint (Draw 4) was first seen as a single incidence in Draw 3. By Draw 4, AR+ cells had once again become a substantial fraction of the population, albeit with a significantly altered CNV profile (Figure 3). We thus infer that Clone C was selected as a drug-resistant subclone from one of the initially depleted metastatic sites. That the early and late stage clones are clearly related and stem from the same lineage is evident from the frequency graphs in Figure 3B, showing that most events are maintained and have identical boundaries. Several other events, however, are either new, deletions on 1q, 8q, and 15q and gains of 3p, 15p, and complex rearrangement of 8q involving a separate amplification of a narrow region containing MYC, or are more frequent in the late stage cells. The co-occurrence of MYC amplification along with re-emergence of AR protein expression and AR amplification may have important therapeutic implications as c-Myc expression confers androgen-independent growth. Koh et al., Genes Cancer 1: 617-628 (2010). While c-Myc has proved a difficult therapeutic target, strategies which target key metabolic and other changes downstream of c-Myc activation are being investigated in many clinical trials. Li and Simon, Clin Cancer Res. (2013). Our data suggests that co-targeting of c-Myc along with AR may provide an approach to delay or prevent the emergence of resistance to abiraterone acetate and other androgen-targeting agents.

Through this selective process, the population of AR negative, pseudodiploid cells remained a significant fraction of cytokeratin positive cells. The presence of these phenotypically (Figure 1A) and genotypically (Figure 3A) distinct cytokeratin positive cells raises the question of their origin. Previous studies have consistently identified cells in primary tumor tissue with similarly unaltered or pseudodiploid CNV profiles. Navin et al., Nature 472: 90-94 (2011). We also cannot exclude that they represent a pre-existing minor population of normal epithelial cells exposed by depletion of the cancer cells in Draw 3, however, that the number of these cells in Draw 3 was comparable to the numbers in Draws 2 and 4 would make that less likely. Alternatively, they may represent tumor associated macrophage lineage cells with phagocytosed intracytoplasmic cytokeratin sloughed off from tumor sites as they are depleted of sensitive cells or a castration resistant stem-like tumor cell population recently described in engrafted prostate tumors and phenotypically characterized as $CK^+$ $AR^-$ cells. Toivanen et al., Sci Transl Med 5: 187 (2013). However, further interrogation of single point mutations combined with protein expression analysis will be required to gain insight into the nature of these cells and their role in tumor progression, if any.

In an era of clinical oncology that is progressively moving towards targeted cancer therapy, approaches that allow for non-invasive monitoring of therapeutic response at both phenotypic and genetic levels are essential. We have chosen to approach this goal through a combined phenotypic and genetic analysis of non-leukocyte circulating nucleated cells, without a pre-selection step that may bias the CTC population. Our method allows us to correlate genomic events with complex phenotypes based on protein expression and cell morphology. Alternative methods, such as sequencing of free DNA from plasma (ctDNA) are also powerful tools and can yield both mutation and copy number information, but only for an admixture of the various cellular components. Murtaza et al., Nature 497: 108-112 (2013). In this case study, we show the remarkable extent and speed of the genomic reorganization as putative-resistant clones emerge at the time of treatment failure. Although, we cannot establish a mechanistic relationship between the large CNV changes and the eventual resistance to abiraterone acetate based on a single patient, it appeared that after 9 weeks of targeted therapy the original CTC population was not completely eliminated and an apparently drug-resistant clone was present. Finally, the integration of data across multiple subjects will open the door for a deeper understanding of the mechanisms and timing of resistance and allow for rationally-designed, personalized treatments based on sequential, combined, or intermittent application of therapeutic agents.

The recitation of a listing of elements in any definition of a variable herein includes definitions of that variable as any single element or combination (or subcombination) of listed elements. The recitation of an embodiment herein includes that embodiment as any single embodiment or in combination with any other embodiments or portions thereof.

From the foregoing description, it will be apparent that variations and modifications can be made to the invention described herein to adopt it to various usages and conditions. Such embodiments are also within the scope of the following claims.

## Claims

1. A method of predicting response to a hormone-directed therapy in a prostate cancer patient comprising

   (a) performing a direct analysis of circulating tumour cells (CTCs) in the context of surrounding nucleated cells present in a blood sample obtained from the patient in the absence of enrichment of the blood sample for CTCs prior to detection thereof, comprising immunofluorescent staining and morphological characterization of nucleated cells in the blood sample to identify and enumerate circulating tumour cells (CTC);
   (b) individually characterizing genotypic, morphometric and protein expression parameters to generate a profile for each of the CTCs, wherein said genotypic parameters comprise copy number variation (CNV) signatures, wherein the CNV signatures comprise gene amplifications or deletions, wherein said gene amplifications comprise genes associated with androgen independent cell growth, wherein said gene comprises v-myc avian myelocytomatosis viral oncogene homolog (MYC);
   (c) predicting response to hormone-directed therapy in the prostate cancer patient based on said profiles of the CTCs;
   (d) repeating steps (a) through (c) at one or more timepoints after initial diagnosis of prostate cancer to sequentially monitor said genotypic, morphometric and protein expression parameters;

and

(e) predicting a response based on comparison of the profiles between the timepoints.

2. The method of claim 1, further comprising identifying clonal lineages of each CTC by genomic analysis.

3. The method of claim 1, wherein said cancer is metastatic castration resistant prostate cancer (mCRPC).

4. The method of claim 1, wherein said hormone directed therapy comprises Androgen Deprivation Therapy (ADT), preferably wherein said ADT is a second line hormonal therapy, more preferably wherein said second line hormonal therapy is selected from the group consisting of abiraterone acetate, ketoconazole and aminoglutethimide.

5. The method of claim 1, wherein the immunofluorescent staining of nucleated cells comprises pan cytokeratin, cluster of differentiation (CD) 45, diamidino-2-phenylindole (DAPI) and androgen receptor (AR).

6. The method of claim 1, wherein said protein expression parameters comprise quantifying protein expression level, preferably wherein said protein expression is AR expression.

7. The method of claim 1, wherein said protein expression parameters comprise subcellular localization of protein expression.

8. The method of claim 1, wherein said morphometric parameters comprise cell shape.

9. The method of claim 1, wherein said response is emergence of resistant disease.

10. The method of claim 9, wherein identification of a resistant CTC correlates with the emergence of resistant disease, preferably wherein the resistant CTC represents a clonal lineage that predominates resistant disease.

11. The method of claim 9, wherein the re-emergence of AR positive CTCs predicts emergence of resistant disease.

12. The method of claim 11, wherein the re-emergence of the AR positive CTCs is accompanied by genomic alterations, or wherein said re-emergence of the AR positive cells is accompanied by morphometric change.

**Patentansprüche**

1. Verfahren zum Vorhersagen der Reaktion auf eine hormongesteuerte Therapie bei einem Prostatakrebspatienten, umfassend

(a) Durchführen einer direkten Analyse von zirkulierenden Tumorzellen (CTCs) in dem Kontext von umgebenden kernhaltigen Zellen, die in einer Blutprobe vorhanden sind, die von dem Patienten in der Abwesenheit von Anreicherung der Blutprobe für CTCs vor deren Nachweis erhalten wurde, umfassend Immunfluoreszenzfärbung und morphologische Kennzeichnung von kernhaltigen Zellen in der Blutprobe zum Identifizieren und Aufzählen von zirkulierenden Tumorzellen (CTC);
(b) individuelles Kennzeichnen von genotypischen, morphometrischen und Proteinexpressionsparametern zum Erzeugen eines Profils für jede der CTCs, wobei die genotypischen Parameter Kopienzahlvariations-(CNV-)Signaturen umfassen, wobei die CNV-Signaturen Genamplifikationen oder -deletionen umfassen, wobei die Genamplifikationen Gene umfassen, die mit androgenunabhängigem Zellwachstum assoziiert sind, wobei das Gen virales onkogenes Homolog von aviärer v-myc-Myelozytomatose (MYC) umfasst;
(c) Vorhersagen der Reaktion auf hormongesteuerte Therapie bei dem Prostatakrebspatienten basierend auf den Profilen der CTCs;
(d) Wiederholen der Schritte (a) bis (c) zu einem oder mehreren Zeitpunkten nach Erstdiagnose von Prostatakrebs, um die genotypischen, morphometrischen und Proteinexpressionsparameter nacheinander zu überwachen; und
(e) Vorhersagen einer Reaktion basierend auf Vergleich der Profile zwischen den Zeitpunkten.

2. Verfahren nach Anspruch 1, ferner umfassend das Identifizieren von klonalen Linien jeder CTC durch Genomanalyse.

3. Verfahren nach Anspruch 1, wobei der Krebs metastasierter kastrationsresistenter Prostatakrebs (mCRPC) ist.

4. Verfahren nach Anspruch 1, wobei die hormongesteuerte Therapie Androgenentzugstherapie (ADT) umfasst, wobei die ADT bevorzugt eine Hormontherapie der zweiten Linie ist, wobei die Hormontherapie der zweiten Linie bevorzugter ausgewählt ist aus der Gruppe bestehend aus Abirateronacetat, Ketoconazol und Aminoglutethimid.

5. Verfahren nach Anspruch 1, wobei die Immunfluoreszenzfärbung von kernhaltigen Zellen Pan-Cytokeratin, Differenzierungscluster (CD) 45, Diamidino-2-phenylindol (DAPI) und Androgenrezeptor (AR) umfasst.

**6.** Verfahren nach Anspruch 1, wobei die Proteinexpressionsparameter das Quantifizieren des Proteinexpressionsniveaus umfassen, wobei die Proteinexpression bevorzugt AR-Expression ist.

**7.** Verfahren nach Anspruch 1, wobei die Proteinexpressionsparameter subzelluläre Lokalisierung der Proteinexpression umfassen.

**8.** Verfahren nach Anspruch 1, wobei die morphometrischen Parameter Zellform umfassen.

**9.** Verfahren nach Anspruch 1, wobei die Reaktion Auftreten einer resistenten Krankheit ist.

**10.** Verfahren nach Anspruch 9, wobei Identifizierung einer resistenten CTC mit dem Auftreten einer resistenten Krankheit korreliert, wobei die resistente CTC bevorzugt eine klonale Linie darstellt, die eine resistente Krankheit dominiert.

**11.** Verfahren nach Anspruch 9, wobei das Wiederauftreten von AR-positiven CTCs das Auftreten einer resistenten Krankheit vorhersagt.

**12.** Verfahren nach Anspruch 11, wobei das Wiederauftreten der AR-positiven CTCs von genomischen Veränderungen begleitet ist oder wobei das Wiederauftreten der AR-positiven Zellen von morphometrischer Veränderung begleitet ist.

## Revendications

**1.** Méthode de prévision de la réaction à une hormonothérapie chez un patient atteint d'un cancer de la prostate comprenant

(a) la réalisation d'une analyse directe des cellules tumorales circulantes (CTC) dans le contexte des cellules nucléées environnantes présentes dans un échantillon sanguin obtenu du patient en l'absence d'enrichissement de l'échantillon sanguin pour les CTC avant leur détection, comprenant une coloration immunofluorescente et une caractérisation morphologique de cellules nucléées dans l'échantillon de sang pour identifier et dénombrer les cellules tumorales circulantes (CTC) ;
(b) la caractérisation individuelle des paramètres génotypiques, morphométriques et d'expression de protéine pour générer un profil pour chacune des CTC, lesdits paramètres génotypiques comprenant des signatures de variation de nombre de copies (CNV), lesdites signatures CNV comprenant des amplifications ou des délétions de gènes, lesdites amplifications de gènes comprenant des gènes associés à la croissance cellulaire indépendante des androgènes, ledit gène comprenant l'homologue de l'oncogène viral de myélocytomatose aviaire v-myc (MYC) ;
(c) la prévision de la réaction à une hormonothérapie chez le patient atteint d'un cancer de la prostate sur la base desdits profils des CTC ;
(d) la répétition des étapes (a) à (c) à un ou plusieurs points temporels après le diagnostic initial du cancer de la prostate pour surveiller de manière séquentielle lesdits paramètres génotypiques, morphométriques et d'expression de protéine ; et
(e) la prévision d'une réaction sur la base de la comparaison des profils entre les points temporels.

**2.** Procédé selon la revendication 1, comprenant en outre l'identification des lignées clonales de chaque CTC par analyse génomique.

**3.** Procédé selon la revendication 1, ledit cancer étant un cancer métastatique de la prostate résistant à la castration (mCRPC).

**4.** Procédé selon la revendication 1, ladite hormonothérapie comprenant une thérapie par privation androgénique (ADT), de préférence ladite ADT étant une thérapie hormonale de seconde ligne, plus préférablement ladite thérapie hormonale de seconde ligne étant choisie dans le groupe constitué par l'acétate d'abiratérone, le cétoconazole et l'aminoglutéthimide.

**5.** Procédé selon la revendication 1, ladite coloration immunofluorescente des cellules nucléées comprenant la pan-cytokératine, le cluster de différenciation (CD) 45, le diamidino-2-phénylindole (DAPI) et le récepteur des androgènes (AR).

**6.** Procédé selon la revendication 1, lesdits paramètres d'expression de protéine comprenant la quantification du niveau d'expression de protéine, de préférence ladite expression de protéine étant l'expression de l'AR

**7.** Procédé selon la revendication 1, lesdits paramètres d'expression de protéine comprenant la localisation subcellulaire de l'expression de protéine.

**8.** Procédé selon la revendication 1, lesdits paramètres morphométriques comprenant la forme de la cellule.

**9.** Procédé selon la revendication 1, ladite réaction étant l'émergence d'une maladie résistante.

10. Procédé selon la revendication 9, l'identification d'une CTC résistante étant en corrélation avec l'émergence d'une maladie résistante, de préférence ladite CTC résistante représentant une lignée clonale qui prédomine une maladie résistante.

11. Procédé selon la revendication 9, la réémergence de CTC positives à l'AR prévoyant l'émergence d'une maladie résistante.

12. Procédé selon la revendication 11, ladite réémergence des CTC positives à l'AR s'accompagnant d'altérations génomiques, ou ladite réémergence des cellules positives à l'AR s'accompagnant d'un changement morphométrique.

**A**

| Time point | | Draw 1<br>Pre Docetaxel | Draw 2<br>Pre Abi-raterone | Draw 3<br>3 wk Abi-raterone | Draw 4<br>9 wk Abi-raterone |
|---|---|---|---|---|---|
| CTCs/ml | AR+ | 167.9 | 37.9 | 7.3 | 30.2 |
| | AR- | 40.7 | 18.9 | 58.7 | 1.1 |
| | Total | 208.6 | 56.8 | 66.0 | 31.3 |

FIG. 1

FIG. 2

FIG. 3

**A**

Colocalization

1.0

0.5

0.0

-0.5

p = 0.00017

Before Abiraterone    After Abiraterone

**B** Before Abiraterone

**D** After Abiraterone

**C**

AR signal intensity

0.6
0.4
0.2
0.0

0.0  0.2  0.4  0.6
DAPI signal intensity

**E**

AR signal intensity

0.6
0.4
0.2
0.0

0.0  0.2  0.4  0.6
DAPI signal intensity

FIG. 4

**Pre-Docetaxel/Bevacizumab-Draw 1**

| | Composite | API | K | AR | CD45 |
|---|---|---|---|---|---|
| AR+ | | | | | |
| HD-CTC AR- | | | | | |

## FIG. 5A

**Pre Abiraterone acetate-Draw 2**

| | Composite | DAPID | CKC | AR | CD45 |
|---|---|---|---|---|---|
| HD-CTC AR+ HD-CTC | | | | | |
| HD-CTC AR- | | | | | |

## FIG. 5B

Three weeks Post Abiraterone acetate- Draw 3

| | Composite | DAPI | CK | AR | CD45 |
|---|---|---|---|---|---|

HD-CTC AR-

HD-CTC AR-

## FIG. 5C

Nine weeks Post Abiraterone acetate-Draw 4

| | Composite | DAPI | CK | AR | CD45 |
|---|---|---|---|---|---|

HD-CTC AR+

HD-CTC AR+

## FIG. 5D

FIG. 6

**FIG. 6**
CONTINUED

FIG. 7

| Cell Nomenclature | Draw | Cluster | AR gene status | AR protein Staining | AR signal intensity | CK signal intensity | Cell Roundness |
|---|---|---|---|---|---|---|---|
| p33164.2.2.CAS0524 | 1 | B | No AR Amp | Negative | 2.54 | 67.22 | 0.925969 |
| p33164.2.2.CAS0520 | 1 | A | AR Amp | Positive | 41.14 | 172.33 | 0.845638 |
| p33164.2.17.CAS0522 | 1 | A | AR Amp | Positive | 84.68 | 100.14 | 0.927069 |
| **p33164.1.5.CAS0498** | **1** | **B** | **No AR Amp** | **Positive** | **11.68** | **70.91** | **0.756361** |
| p33164.2.6.CAS0521 | 1 | A | AR Amp | Positive | 23.27 | 154.74 | 0.80859 |
| p33164.2.20.CAS0523 | 1 | A | AR Amp | Positive | 106.40 | 65.24 | 0.966375 |
| p33164.1.12.CAS0501 | 1 | A | AR Amp | Positive | 20.98 | 80.76 | 0.963558 |
| p33164.2.21.CAS0525 | 1 | A | AR Amp | Positive | 69.59 | 42.92 | 0.846964 |
| p33164.1.13.CAS0502 | 1 | A | AR Amp | Positive | 32.78 | 68.95 | 0.874024 |
| p33164.2.22.CAS0526 | 1 | A | AR Amp | Positive | 132.3 | 51.96 | 0.87912 |
| ARPred.1.2.CAS0568 | 2 | A | AR Amp | Positive | 11.00 | 114.09 | 0.806197 |
| ARPred.1.3.CAS0569 | 2 | A | AR Amp | Positive | 46.05 | 32.61 | 0.642296 |
| **ARNred.1.2.CAS0589** | **2** | **A** | **AR Amp** | **Negative** | **1.59** | **160.01** | **0.893199** |
| p33164.5.2.CAS0533 | 2 | A | AR Amp | Positive | 84.30 | 42.84 | 0.737126 |
| p33164.5.4.CAS0534 | 2 | A | AR Amp | Positive | 92.47 | 69.28 | 0.729997 |
| p33164.5.6.CAS0535 | 2 | A | AR Amp | Positive | 47.80 | 125.17 | 0.370085 |
| **ARPred.1.4.CAS0570** | **2** | **A** | **AR Amp** | **Negative** | **2.77** | **46.68** | **0.512653** |
| p33164.5.7.CAS0536 | 2 | A | AR Amp | Positive | 90.00 | 49.65 | 0.854095 |
| ARNred.1.5.CAS0592 | 2 | A | AR Amp | Positive | 9.11 | 95.68 | 0.920361 |
| ARNblue.1.1.CAS0595 | 3 | B | No AR Amp | Negative | 1.71 | 18.44 | 0.699269 |
| p33164.6.29.CAS0561 | 3 | B | No AR Amp | Negative | 5.98 | 44.98 | 0.793052 |
| p33164.6.15.CAS0559 | 3 | B | No AR Amp | Negative | -0.17 | 16.89 | 0.700298 |
| p33164.6.14.CAS0560 | 3 | B | No AR Amp | Negative | 2.97 | 33.33 | 0.795308 |
| p33164.6.32.CAS0562 | 3 | B | No AR Amp | Negative | 1.92 | 77.42 | 0.464112 |
| ARNblue.1.6.CAS0599 | 3 | B | No AR Amp | Negative | 3.82 | 98.88 | 0.886125 |
| p33164.6.34.CAS0563 | 3 | B | No AR Amp | Negative | 2.23 | 43.11 | 0.633107 |
| p33164.6.36.CAS0564 | 3 | B | No AR Amp | Negative | 0.33 | 14.63 | 0.804486 |
| **p33164.6.37.CAS0565** | **3** | **C** | **AR Amp** | **Negative** | **0.92** | **34.28** | **0.614988** |
| ARNblue.1.5.CAS0598 | 3 | B | No AR Amp | Negative | 1.22 | 33.02 | 0.673803 |
| ARNblue.1.8.CAS0601 | 3 | B | No AR Amp | Negative | 1.25 | 22.36 | 0.560946 |
| **ARNblue.1.7.CAS0600** | **3** | **A** | **AR Amp (low)** | **Negative** | **0.53** | **9.302** | **0.422198** |
| p33164.7.22.CAS0632 | 4 | A | AR Amp | Positive | 37.82 | 50.80 | 0.878598 |
| ARPblack.1.2.CAS0603 | 4 | C | AR Amp | Positive | 16.80 | 108.81 | 0.932083 |
| ARPblack.3.2.CAS0618 | 4 | C | AR Amp | Positive | 85.29 | 116.14 | 0.512847 |
| ARPblack.1.4.CAS0613 | 4 | C | AR Amp | Positive | 57.45 | 27.05 | 0.68 |
| p33164.7.27.CAS0634 | 4 | A | AR Amp | Positive | 9.11 | 37.54 | 0.936407 |
| p33164.7.28.CAS0636 | 4 | A | AR Amp | Positive | 19.09 | 27.47 | 0.932493 |
| **p33164.7.30.CAS0638** | **4** | **A** | **AR Amp** | **Negative** | **0.65** | **19.72** | **0.903884** |
| ARPblack.3.3.CAS0619 | 4 | C | AR Amp | Positive | 38.64 | 59.20 | 0.901781 |
| p33164.7.37.CAS0642 | 4 | C | AR Amp | Positive | 38.76 | 16.79 | 0.858456 |
| ARPblack.1.6.CAS0616 | 4 | C | AR Amp | Positive | 9.56 | 15.50 | 0.835043 |

# FIG. 8

| exon# | AA# | mutation | Primary tumor #1 % Mutant Read | Primary tumor #2 % Mutant Read | Cluster A fraction mutant cells | Cluster B fraction mutant cells | Cluster C fraction mutant cells |
|---|---|---|---|---|---|---|---|
| exon2 | 543 | GCC-GCG | | | 1/12 | 0 | 0 |
| | 544 | AGG-AAG | | | 1/12 | 0 | 0 |
| | 549 | CCC-ACC | | | 1/12 | 0 | 0 |
| | 555 | CAC-CCC | 12% | 12% | 0 | 0 | 0 |
| | 627 | GTG-GTA | | | 3/12 | 0 | 0 |
| | 649 | ACC-GCC | 5% | 4% | 1/12 | 0 | 0 |
| exon4 | 653 | ACT-GCT | | | 0 | 1/5 | 0 |
| | 653 | ACT-ACC | 17% | | 0 | 0 | 0 |
| | 654 | GAG-GGG | 21% | | 0 | 1/5 | 0 |
| | 655 | GAG-GGG | 7% | | 0 | 0 | 0 |
| | 656 | ACA-GCA | 18% | 2% | 0 | 0 | 0 |
| | 656 | ACA-ACG | 4% | | 0 | 0 | 1/10 |
| | 682 | GAG-GAA | 17% | 4% | 1/12 | 0 | 0 |
| exon6 | 790 | CAC-CG/CC | 4% | 5% | 6/12 | 0/5 | 7/10 |
| | 795 | TTT-TTC | 3% | 2% | 7/12 | 1/5 | 7/10 |
| | intron | C---T | | | 7/12 | 0/5 | 7/10 |
| exon7 | 823 | AAA-AGA | 3% | 8% | 0/12 | 1/5 | 0.1 |
| exon8 | 897 | GCA-GTA | 3% | 4% | 5/12 | 4/5 | 1/10 |

# FIG. 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20130171642 A
- US 20120276555 A
- WO 2012103025 A
- WO 2015116828 A

**Non-patent literature cited in the description**

- **SIEGEL et al.** *CA Cancer J Clin. 2014,* 2014, vol. 64 (1), 9-29
- **MIYAMOTO et al.** *Cancer Discovery,* October 2012, vol. 2 (11), 995-1003
- **STOTT et al.** *Science Translational Medicine,* March 2010, vol. 2 (25), 111-120
- **ATTARD et al.** *Current Opinion in Genetics & Development,* November 2010, vol. 21 (1), 50-58
- **HALIR ; FLUSSER.** *Proceeding of International Conference in Central Europe on Computer Graphics, Visualization and Interactive Digital Media,* 1998, 125-132
- **MARRINUCCI et al.** *Phys Biol,* 2012, vol. 9, 016003
- **AR. LAZAR et al.** *Phys Biol,* 2012, vol. 9, 016002
- **NAVIN et al.** *Nature,* 2011, vol. 472, 90-94
- **BASLAN et al.** *Nat Protoc,* 2012, vol. 7, 1024-1041
- **HALIR ; FLUSSER.** Proceeding of International Conference in Central Europe on Computer Graphics, Visualization and Interactive Digital Media. 1998, 125-132
- **MARRINUCCI D. et al.** *Phys. Biol.,* 2012, vol. 9, 016003
- **NIEVA et al.** *Phys Biol,* 2012, vol. 9, 016004
- **PRICE ; NEWMAN.** Principles and Practice of Immunoassay. Grove's Dictionaries, 1997
- **GOSLING.** Immunoassays: A Practical Approach. Oxford University Press, 2000
- **SELF et al.** *Curr. Opin. Biotechnol.,* 1996, vol. 7, 60-65
- **JOHN R. CROWTHER.** The ELISA Guidebook. Humana Press, 2000
- An Introduction to Radioimmunoassay and Related Techniques. Elsevier Science, 1995
- **COLIGAN.** *Current Protocols in Immunology,* 1991
- **HARLOW ; LANE.** Antibodies: A Laboratory Manual. 1988
- **GODING.** Monoclonal Antibodies: Principles and Practice. 1986
- **BOX ; COX.** *Royal Stat. Soc., Series B,* 1964, vol. 26, 211-246
- **LAZAR et al.** *Phys Biol,* 2012, vol. 9, 016002
- **NAIR et al.** *PLoS One,* 2013, vol. 8, e67733
- **MEYER et al.** *Nucleic Acids Res,* 2013, vol. 41, D64-69
- **TEAM RC.** *R: A Language and Environment for Statistical Computing,* 2012
- **TAYLOR et al.** *Cancer Cell,* 2010, vol. 18, 11-22
- **GOATLY et al.** *Mod Pathol,* 2008, vol. 21, 902-911
- **GARRAWAY et al.** *Nature,* 2005, vol. 436, 117-122
- **KOIVISTO et al.** *Cancer Res,* 1997, vol. 57, 314-319
- **MIYAMOTO et al.** *Cancer Discov,* 2012, vol. 2, 995-1003
- **KOH et al.** *Genes Cancer,* 2010, vol. 1, 617-628
- **MOSTAGHEL et al.** *Clin Cancer Res,* 2011, vol. 17, 5913-5925
- **CHAN et al.** *J Biol Chem,* 2012, vol. 287, 19736-19749
- **MAGBANUA et al.** *BMC Cancer,* 2012, vol. 12, 78
- **HEITZER et al.** *Genome Med,* 2013, vol. 5, 30
- **NAVIN et al.** *Genome Res,* 2010, vol. 20, 68-80
- **GERLINGER et al.** *N Engl J Med,* 2012, vol. 366, 883-892
- **ALMENDRO et al.** *Cancer Res,* 2014, vol. 74, 1338-1348
- **SEQUIST et al.** *Sci Transl Med,* 2011, vol. 3
- **SHI et al.** *Cancer Discov,* 2014, vol. 4, 80-93
- **LI ; SIMON.** *Clin Cancer Res.,* 2013
- **TOIVANEN et al.** *Sci Transl Med,* 2013, vol. 5, 187
- **MURTAZA et al.** *Nature,* 2013, vol. 497, 108-112